# EUROPEAN PATENT SPECIFICATION

(11) **EP 3 648 587 B1**
(45) Date of publication and mention of the grant of the patent: **29.05.2024**
(21) Application number: 18743421.2
(22) Date of filing: 05.07.2018
(51) Int. Cl.: A01K 67/027, C12N 15/85

(54) **CELLS, VERTEBRATES, POPULATIONS & METHODS**
ZELLEN,VERTEBRATEN,POPULATIONEN UND VERFAHREN
CELLULES, VERTEBRATES,POPULATIONS ET METHODES

(30) Priority: 07.07.2017 GB 201710984
(43) Date of publication of application: 13.05.2020
(73) Proprietor: Kymab Limited, Cambridge, Cambridgeshire CB33 3AT (GB)
(72) Inventor: LEE, E-Chiang, Cambridge, Cambridgeshire CB22 3AT (GB); WANG, Wei, Cambridge, Cambridgeshire CB22 3AT (GB); BLACKWOOD, John Kenneth, Cambridge CB22 3AT (GB); MAGLIOZZI, Roberto, Cambridge CB22 3AT (GB); WOOD, Andrew, Cambridge, CB22 3AT (GB)
(74) Representative: CMS Cameron McKenna Nabarro Olswang LLP
(86) International application number: PCT/EP2018/068309
(87) International publication number: WO 2019/008123

(56) References cited:
- EP-A1- 2 147 594
- WO-A1-2014/130690
- WO-A2-2006/117699
- US-A1- 2006 015 957
- US-A1- 2011 195 454
- US-A1- 2012 021 409
- US-A1- 2013 263 292
- US-A1- 2018 298 112
- MATTHEW A. INLAY ET AL: "Critical roles of the immunoglobulin intronic enhancers in maintaining the sequential rearrangement of IgH and Igk loci", THE JOURNAL OF EXPERIMENTAL MEDICINE, vol. 203, no. 7, 10 July 2006 (2006-07-10) , pages 1721-1732, XP055516424, US ISSN: 0022-1007, DOI: 10.1084/jem.20052310
- DIAZ M ET AL: "Somatic immunoglobulin hypermutation", CURRENT OPINION IN IMMUNOL, ELSEVIER, OXFORD, GB, vol. 14, no. 2, 1 April 2002 (2002-04-01), pages 235-240, XP004340052, ISSN: 0952-7915, DOI: 10.1016/S0952-7915(02)00327-8
- T. PERLOT ET AL: "Elucidation of IgH intronic enhancer functions via germ-line deletion", PROCEEDINGS OF THE NATIONAL ACADEMY OF SCIENCES OF THE UNITED STATES OF AMERICA, vol. 102, no. 40, 4 October 2005 (2005-10-04), pages 14362-14367, XP055516419, ISSN: 0027-8424, DOI: 10.1073/pnas.0507090102
- WINTER D B ET AL: "Insertion of 2 kb of bacteriophage DNA between an immunoglobulin promoter and leader exon stops somatic hypermutation in a kappa transgene", MOLECULAR IMMUNOLOGY, PERGAMON, GB, vol. 34, no. 5, 1 April 1997 (1997-04-01), pages 359-366, XP002556634, ISSN: 0161-5890, DOI: 10.1016/S0161-5890(97)00073-4 [retrieved on 1997-10-10]
- ODEGARD VALERIE H ET AL: "Targeting of somatic hypermutation", NATURE REVIEWS IMMUNOLOGY, NATURE PUBLISHING GROUP UK, LONDON, vol. 6, no. 8, 1 August 2006 (2006-08-01), pages 573-583, ISSN: 1474-1733, DOI: 10.1038/NRI1896
- BETZ A G ET AL: "Elements regulating somatic hypermutation of an immunoglobulin @k gene: Critical role for the intron enhancer/matrix attachment region", CELL, ELSEVIER, AMSTERDAM NL, vol. 77, no. 2, 22 April 1994 (1994-04-22) , pages 239-248, ISSN: 0092-8674, DOI: 10.1016/0092-8674(94)90316-6 [retrieved on 1994-04-22]
- BETZ A G ET AL: "Elements regulating somatic hypermutation of an immunoglobulin @k gene: Critical role for the intron enhancer/matrix attachment region", CELL, ELSEVIER, AMSTERDAM NL, vol. 77, no. 2, 22 April 1994 (1994-04-22) , pages 239-248, XP024245265, ISSN: 0092-8674, DOI: 10.1016/0092-8674(94)90316-6 [retrieved on 1994-04-22]
- ODEGARD VALERIE H ET AL: "Targeting of somatic hypermutation", NATURE REVIEWS IMMUNOLOGY, NATURE PUBLISHING GROUP UK, LONDON, vol. 6, no. 8, 1 August 2006 (2006-08-01), pages 573-583, XP002556635, ISSN: 1474-1733, DOI: 10.1038/NRI1896

## Description

### FIELD OF THE INVENTION

The invention relates to cells and non-human vertebrates for producing antibody chains, in particular for use in producing multi-specific antibodies useful for therapy or diagnosis.

### BACKGROUND

Antibodies are a rapidly growing drug class. A typical 4-chain antibody contains two identical heavy chains and two identical light chains, which form a molecule with bivalent and monspecific antigen binding features. The bispecific antibody is a new class of drug which can bind two different antigens or two different epitopes of the same antigen at the same time. There are several applications of bispecific antibodies for disease treatment. The most widely used application of this concept is in cancer immunotherapy, where bispecific antibodies engage cytotoxic T cells and target to tumour cells to be destroyed. They can also be applied to simultaneously block to two targets (Klein, C et al. MABs. 2016. 8: 1010-20.), or act as a cofactor for an enzymatic pathway (Kitazawa, T. et al. Nat Med. 2012. 18: 1570-4.). Two bispecific antibodies, catumaxomab (anti-EpCAM × anti-CD3) and blinatumomab (anti-19 × anti-CD3) have been approved for therapy, and several additional bispecific antibodies are currently in clinical development. There are five different structural groups for bispecific antibodies: (i) bispecific IgG (BsIgG) (ii) IgG appended with an additional antigen-binding moiety (iii) BsAb fragments (iv) bispecific fusion proteins and (v) BsAb conjugates (Spiess, C. et al. Mol. Immunol. 2015. 67:95-106.). Among them, BsIgG is a bispecific antibody with molecular weight close to a IgG molecule. It usually has similar biological and biophysical features to a typical IgG. Production of BsIgG by co-expression of the two antibodies in cells is a highly challenge because of the low yield of desired BsIgG and the difficulty to remove closely related mispaired IgG molecules. Two heavy chains can from homodimers as well as desired heterodimers. In addition, light chains can mispair with non-cognate heavy chains. Consequently, coexpression of two antibodies results in nine unwanted IgG species and only one desired BsIgG due to the heavy chain pairing and light chain pairing problems.

Engineering heavy chains to favour the heterodimerization has been demonstrated by the "knob-into-hole" technology (Ridgway, J.B. et al. Protein Eng. 1996. 9: 617-21.; Atwell, S. et al. J. Mol. Biol. 1997. 270: 26-35.) or the "charge pairing" technology (Gunasekaran, K. et al. J. Biol. Chem. 2010. 285: 19637-46.; Strop, P. et al. J. Mol. Biol. 2012. 420: 204-19.). These technologies aim to resolve the heavy chain pairing problem. The light chain pairing problem is obviated by expression of antibodies using common light chains (Merchant, M. et al. Nat. Biotechnol. 1998. 16: 677-81.). By applying the "knob-into-hole" or "charge pairing", and common light chain technologies, coexpression of one light chain and two heavy chains results in one dominant BsIgG and three minor mispairing species. The knobs-into-holes technology is described in US5,731,168 and WO98/50431. Purification steps using cation exchange (Sampei, Z. et al. PLoS One. 2013. 8: e57479.) or differential protein A binding (Smith, E.J. et al. Sci. Rep. 2015. 5: 17943.) have been applied to isolate this dominant expressed BsIgG.

It is challenging to identify a common light chain for BsIgG. Forced pairing of non-cognate heavy and light chains usually results in low yield of expression or instability of molecules. Light chain shuffling between two cognate paired light chains has been used to identify the common light chain (Sampei, Z. et al. PLoS One. 2013. 8: e57479.). This, however, relies on tedious steps and a lot of effort, and the outcome is also uncertain. Compared to this method, the "common light chain transgenic" technology is promised to be more straightforward and simple, relying on *in vivo* selection to generate the natural pairing molecules. In these applications, mice engineered to contain only one chimeric light chain-encoding sequence (human variable and mouse constant regions) are used to identify antibodies sharing the similar but not necessarily identical light chain because the inserted rearranged light chain sequence is still susceptible to somatic hypermutation (SHM). The inserted light chain choice is also limited as it relies on germline transmitted mice with one light chain sequence insertion. WO2004/106375, WO2009/1577771, EP2147594 and WO2014/160179 discuss techniques relevant to production of bispecific antibodies and common light chain mice.

EP2147594 discloses transgenic, non-human animals comprising a nucleic acid encoding an immunoglobulin light chain, whereby the immunoglobulin light chain is human, human-like, or humanized. The nucleic acid is provided with a means that renders it resistant to DNA rearrangements and/or somatic hypermutations.

### STATEMENT OF INVENTION

The invention is as defined in the accompanying claims.

The inventors realised that the provision of a V domain coding variable region sequence 3' of the intronic enhancer in an antibody chain locus minimises SHM of the V region, which allows the invention to be used to identify and use true common light chains, VL domains and VH domains that can pair with these to produce antigen binding sites.

Further exemplification is provided below by way or worked experiments and data.

### BRIEF DESCRIPTION OF THE DRAWINGS

**Figure 1**
   A schematic of the targeting strategy of generating a common light chain allele. The mouse κ intronic enhancer and mouse κ constant region (4.6 kb) in mouse ES cells were replaced by a synthesised DNA fragment of 6.8 kb, which contains a EF1α-Puro-2A-EGFP cassette, the natural promoter and signal peptide of human Vλ 3-21, mouse κ intronic enhancer, as well as a recombined human common lambda light chain allele from an antibody that specifically binds Target X.
**Figure 2**
   A schematic of the sorting strategy to isolate spleen B cells that express antibodies with a human λ light chain and specifically bind human Target X from a common light chain knock-in chimera mouse. The sorting strategy involved lymphocyte section, followed by single cell selection, followed by live cell selection, followed by CD19⁺ B-cell selection and finally human lambda positive and antigen (Target X) positive cell selection. SSC: side scatter. FSC: forward scatter. SSC-A: side scatter area.
**Figure 3**
   Sorting plots showing the isolation of B cells that express antibodies with a human λ light chain and specifically bind human Target X from a common light chain knock-in chimera mouse. Graphs A-F are sorting plots and show the flow of sorting. The cells selected in the first sort were used for the subsequent sort and the selection process continued. A. Sorting plot selecting lymphocytes. SSC-A (side scatter area) versus FSC-A (forward scatter area). B. Sorting plot selecting single cells. FSC-W (forward scatter width) versus FSC-A (forward scatter area). C. Sorting plot selecting single cells. SSC-W (side scatter width) versus SSC-A (side scatter area). D. Sorting plot selecting live cells. SSC-A (side scatter area) versus live/dead 7-AAD. E. Sorting plot selecting CD19⁺ B-cells. Y-axis indicates markers used versus CD19-PB. F. Sorting plot of B-cells that express human lambda light chain and specifically bind human Target X. Human Target X antigen labelled with AF647 versus human lambda light chain labelled with PE.
**Figure 4**
   VL regions of the light chains recovered by NGS essentially all carry the same 6 non-germline mutations as the knock-in common light chain allele.
**Figure 5**
   Chimera mice generated from Rag^{-/-} blastocysts micro-injected with targeted ES cells (targeted Rag^{-/-}) had similar total viable spleen cell number compared to wild-type mice (Wild-Type).
**Figure 6**
   Chimera mice generated from Rag^{-/-} blastocysts micro-injected with targeted ES cells (targeted Rag^{-/-}) had similar CD19-postive, B220-positve B-cell number compared to wild-type mice (Wild-Type).
**Figure 7**
   Percentage CD19-positive, B220-positive splenic B-cell population in chimera mice generated from Rag^{-/-} blastocysts micro-injected with targeted ES cells (targeted Rag^{-/-}) was similar to wild-type mice (Wild-Type).
**Figure 8**
   Percentage CD19-positive, B220-positive splenic B-cell population in chimera mice generated from Rag^{-/-} blastocysts micro-injected with targeted ES cells (targeted Rag^{-/-}) was similar to wild-type mice (Wild-Type).
**Figure 9**
   Percentage antigen positive IgG B-cell population in chimera mice generated from Rag^{-/-} blastocysts micro-injected with targeted ES cells (targeted Rag^{-/-}) was similar to wild-type mice (Wild-Type).

### DETAILED DESCRIPTION

Aspects herein may be expressed in terms of the non-human vertebrate being a mouse, but it is to be understood that rodents, rats, rabbits or any other non-human vertebrate may be suitable for performing the present invention.

The inventors realised that the provision in an antibody light or heavy chain locus of a V domain coding variable region sequence 3' of the intronic enhancer in an antibody chain locus minimises SHM of the V region, which allows the invention to be used to identify true common light or heavy chains. In some embodiments, the locus is a modified version of an endogenous antibody locus of a wild-type or transgenic vertebrate cell or non-human vertebrate. In other embodiments, the locus is not at an endogenous antibody chain locus, but instead is comprised by a locus inserted at Rosa 26 or another location where the locus is capable of expressing antibody chains. For example, the locus of the disclosure is comprised by a randomy inserted transgene in the cell or vertebrate genome. By minimising or eliminating SHM of the V region, a single (ie, "common") light chain is more likely to be expressed by the cell or such cells comprised by the vertebrate. When SHM is eliminated the variable region expresses only a single type of VL domain which may pair with different VH domains expressed by the vertebrate, thereby allowing screening and identification of one or more VH domains that may functionally pair with the VL to form VH/VL binding sites for an antigen or epitope. Even if some mutation may occur, the invention restricts the possibility of this so that the purity of VL domains produced by the same V-J recombination is likely to be such that a single rearranged VL by far predominates (eg, at a level of at least 95% or 100% as described further below). Usefully, therefore, the expression of a desired common light chain can be pre-determined (eg, by providing a known variable region (or light chain) sequence in the locus or transgene, such as a known rearranged variable region (or light chain) sequence encoding a VL (or light chain) of a known antibody that can specifically bind to a predetermined antigen or epitope. Advantageously, the ability in embodiments to use a light chain sequence (eg, a human rearranged VκJκCκ of a predetermined human antibody of known binding specificity and/or affinity for an antigen or epitope) is useful as pairings and selection *in vivo* in the vertebrate of the invention is then possible in the context of the complete human light chain that will be used in a final antibody product (eg, a bispecific antibody medicament).

Optionally, the common light chain comprises or consists of the amino acid sequence of SEQ ID NO: 1, or an amino acid sequence that is at least 70, 75, 80, 85, 90, 95, 96, 97, 98 or 99% identical thereto. Optionally, the variable domain of the common light chain comprises or consists of the variable domain of the light chain sequence of SEQ ID NO: 1, optionally with 5 or less, 4 or less, 3 or less, 2 or 1 amino acid change. Each change can be an amino acid deletion, substitution or addition. In an example, the sequence of the variable domain is determined by Kabat numbering. In an example, the sequence of the variable domain is determined by IMGT numbering.

An example of an *in vivo* approach using the chimaeric mouse (or other no-human vertebrate) to identify common light chains for muti- or bi-specific antibodies is provided, wherein B cells from the mouse are derived from ES cells with rearranged light chain loci and expressing an immunoglobulin light chain repertoire with a single or highly limited number of light chain variable domains. The chimeric mouse is, for example, generated from wild type, RAG knock-out or IgH knock-out mouse blastocysts injected with mouse ES cells carrying the knock-in rearranged human light chain encoding sequence and an unrearranged human or endogenous IgH locus. The DNA sequence of the rearranged human light chain is inserted such that it may be free of somatic hypermutation (SHM). Methods for making such a chimaeric mouse or other non-human vertebrate expressing common light chains are provided. Methods for identify antigen-specific common light chain antibodies useful as bispecific antibodies (eg, for cancer therapy in a human) are provided, as are such antibodies *per se* and cells expressing these.

A particular example work flow may be:-
1. Obtaining a first antibody comprising first human VH and VL domains, wherein the antibody specifically binds to a first predetermined antigen; or obtaining nucleotide sequences encoding said VL or the light chain of the antibody;
2. Obtaining a transgenic mouse ES cell comprising an IgH locus (in heterozygous or homozygous form), wherein the IgH locus comprises a humanised variable region for expressing a plurality of different human VH domains;
3. Inserting a nucleotide sequence encoding the light chain of the antibody or the VL thereof into the genome of the ES cell, wherein the insertion is between the Eiκ and Cκ of a kappa locus allele of the ES cell genome;
4. Optinally modifying the other kappa locus allele so that it is not capable of expressing kappa light chains, or modifying the allele whereby a nucleotide sequence encoding the light chain of the first or a second antibody or the VL thereof is inserted between the Eiκ and Cκ of said other kappa locus allele;
5. Developing the ES cell into a mouse (eg, by implanting the ES cell into a recipient blastocyst or pre-morula embryo (eg, a RAG and/or IgH knock-out blastocyst or pre-morula embryo) and implanting the blastocyst or pre-morula embryo into a pseudopregnant female mouse, whereby one or more pups are birthed from the female), wherein the mouse or pup(s) so developed comprises at least one kappa locus that is capable of expressing the light chain or VL, wherein the light chain or VL pair with heavy chains or VH expressed by the mouse to form VH/VL antigen binding sites;
6. Optionally obtaining progeny mice, wherein the progeny each comprise at least one kappa locus that is capable of expressing the light chain or VL, wherein the light chain or VL pair with heavy chains or VH expressed by the progeny mouse to form VH/VL antigen binding sites;
7. Immunising the mouse of step 5 or a said progeny mouse with a second antigen, wherein the first and second antigens are different;
8. Obtaining from an immunised mouse of step 7 (eg, using PCR of DNA of a B-cell and/or hybridoma technology of a B-cell of an immunised mouse of step 7) a nucleotide sequence encoding a VH (second VH) that pairs with the VL (or a VH of a heavy chain that pairs with the VL of the light chain), wherein the second VH and VL form an antigen binding site that specifically binds to the second antigen;
9. Optionally inserting a nucleotide sequence encoding the first VH, a nucleotide sequence encoding the second VH, and a nucleotide sequence encoding the VL into one or more expression vectors or into the genome of one or more host cells, each V nucleotide sequence being in operable connection 5' of an antibody first CH, second CH or CL region respectively for expression of first antibody heavy chains, second antibody heavy chains or antibody light chains respectively; optionally wherein the first and second CH regions encode first and second CH domains that are mutated human CH domains that pair together (eg, using charge pair mutation or knob-in-hole mutation) and/or wherein the first and second chains have different pl (eg, separated by 0.5-1 pl unit);
10. Optionally obtaining host cells of step 9 and expressing the heavy and light chains in the host cells, whereby bi-specific antibodies are produced each comprising a first heavy chain/light chain pair comprising a VH/VL binding site that is capable of specifically binding to the first antigen, and a second heavy chain/light chain pair comprising a VH/VL binding site that is capable of specifically binding to the second antigen; and
11. Optionally isolating said bi-specific antibodies.

In an alternative, first and second eptiopes of an antigen are used instead of first and second antigens. In an embodiment, the method comprises steps 1 to 8; 1 to 9; 1 to 10 or 1 to 11. In an example, there is provided a method for obtaining a bispecific antibody, the method commencing at step 7. In an example, there is provided a method for obtaining a bispecific antibody, the method commencing at step 8. In an example, there is provided a method for obtaining a bispecific antibody, the method commencing at step 9. In an example, there is provided a method for obtaining a bispecific antibody, the method commencing at step 10. In an example, there is provided a method for obtaining a bispecific antibody, the method commencing at step 11.

In an example, the light chain repertoire of each mouse used in step 7 is at least 95, 96, 97, 98 or 99% pure (or 100% pure) for the VL. This can be determined, for example, by obtaining a B-cell sample (eg, spleen and/or bone marrow B-cell sample) from the mouse, obtaining VL-encoding nucleotide sequences thereof (eg, mRNA or cDNA) and carrying out next-generation sequencing (NGS) to determine the percentage of the VL (and any other VL species) in the sampled nucleotide sequences. The skilled person will be familiar with routine NGS techniques. Thus, for example, herein "95% pure" in respect of the VL means that the repertoire (eg, a sampled repertoire of the mouse) comprises sequences (eg, mRNA, cDNA or DNA) encoding said predetermined VL in a proportion in the repertoire or sample of 95% or higher, eg, as determined by NGS. In an embodiment, all of (or substantially all of) the VL domains expressed by the mouse are identical to the VL of the antibody of step 1. This may be advantageously be achieved due to the positioning according to the invention of the VL-encoding sequence 3' of the intronic enhancer, such as between this enhancer and the constant region.

In an alternative, the VL-encoding variable region sequence (eg, in steps 3 and 4 above) is an insert in the lambda locus allele(s) of the cell or mouse between mouse lambda 2-4 and Cλ or between mouse lambda 4-10 enhancer and Cλ. Additionally, in an embodiment the VL-encoding variable region sequence is an insert in the kappa locus allele(s) of the cell or mouse between Eiκ and Cκ.

In an alternative, only one of the lambda locus alleles comprises a said insert; and only one of the kappa locus alleles comprises a said insert.

The VL-encoding variable region inserts in the lambda and kappa, or in the 2 lambda or in the 2 kappa alleles may be the same or different (ie, encode the same or different VLs, such as VLs of 2 different antibodies of predetermined specificity for the same or different antigens).

In an embodiment, first and second mouse (or other non-human vertebrate, such as rat) ES cells are modified to comprise first and second loci of the disclosure, eg, each cell being modified according to steps 1 to 4, but wherein the VL-encoding nucleotide sequence inserts in the cells are different (ie, encode different VL domains, such as VL domains of different antibodies of different antigen or epitope specificity). The first and second ES cells (or progeny thereof) are implanted into the same blastocyst or pre-morula embryo and a mouse (or progeny thereof) developed, wherein the mouse or progeny express said different VL domains for pairing with VH domains (eg, human VH) expressed by the mouse or progeny.

Advantageously, a first generation mouse (ie, grown directly from the ES cell-implanted blastocyst or pre-morula herein) is useful as it can be immunised without the need for further breeding to subsequent generation mice. This saves time and complexity of breeding. It may be desirable, for example, for the blastocyst or pre-morula that receives the ES cell implant to comprise a knock-out of its IgH loci or be a RAG knock-out, as this forces all productive B-cells produced in the first generation mouse (or subsequent generation mice) to be ES cell derived, thereby ensuring that all productive B-cells comprise one or more loci of the invention.

Optionally, thus generally it may advantageously not be necessary to obtain germline transmission of the loci of the invention in non-human vertebrate progeny or lineages, instead it is possible to work with chimaera vertebrates grown from the blastocyst or pre-morula embryo which provides significant time advantages over prior art techniques where germline transmission of light chain allele modifications is described. Thus, in one embodiment, a mouse or vertebrate of the invention is a chimeaera comprising a first plurality of cells of the invention and a second plurality of cells that are not according to the invention (eg, wild-type or transgenic cells that do not comprise a locus of the invention). Optionally, the chimaera may comprise a third plurality of cells according to the invnetion comprising loci according to the invention but that are capable of expressing V domains that are different from the V domains expressed by the loci of the invention of the first plurality of cells. Thus, it is possible to develop a chimaeric vertebrate that can express first and second VL domains that are different but which are predetermined as they are expressed from loci of the invention. It may be advantageous to knock out expression of one or more light and/or heavy chain loci of the second plurality of cells so that heavy and light chains in the vertebrate are only contributed by the first and optional third plurality of cells. In addition or alternatively, the second plurality of cells may have a RAG (eg, RAG-1 and/or RAG-2) knock-out to eliminate antibody chain contribution from such cells in the vertebrate.

A V domain or binding site that "specifically binds to" or is "specific for" a particular antigen or epitope is one that binds to that particular antigen or epitope without substantially binding to other antigens or epitopes. For example, binding to the antigen or epitope is specific when the antibody binds with a K_{D} of 1mM or less, eg, 100 µM or less, 10 µM or less, 1 µM or less, 100 nM or less, eg, 10 nM or less, 1 nM or less, 500 pM or less, 100 pM or less, or 10pM or less. The binding affinity (K_{D}) can be determined using standard procedures as will be known by the skilled person, eg, binding in ELISA and/or affinity determination using surface plasmon resonance (eg, Biacore^{™}, Proteon^{™} or KinExA^{™} solution phase affinity measurement which can detect down to fM affinities (Sapidyne Instruments, Idaho)). In one embodiment, the surface plasmon resonance (SPR) is carried out at 25°C. In another embodiment, the SPR is carried out at 37°C. In one embodiment, the SPR is carried out at physiological pH, such as about pH7 or at pH7.6 (eg, using Hepes buffered saline at pH7.6 (also referred to as HBS-EP)). In one embodiment, the SPR is carried out at a physiological salt level, eg, 150mM NaCl. In one embodiment, the SPR is carried out at a detergent level of no greater than 0.05% by volume, eg, in the presence of P20 (polysorbate 20; eg, Tween-20TM) at 0.05% and EDTA at 3mM. In one example, the SPR is carried out at 25°C or 37°C in a buffer at pH7.6, 150mM NaCl, 0.05% detergent (eg, P20) and 3mM EDTA. The buffer can contain 10mM Hepes. In one example, the SPR is carried out at 25oC or 37oC in HBS-EP. HBS-EP is available from Teknova Inc (California; catalogue number H8022).

In an example, the affinity is determined using SPR by
1. Coupling anti-mouse (or other relevant non-human vertebrate, to match the C region of an antibody for example) IgG (eg, Biacore BR-1008-38) to a biosensor chip (eg, GLM chip) such as by primary amine coupling;
2. Exposing the anti-mouse IgG (non-human vertebrate antibody) to a test IgG antibody or heavy chain to capture test antibody on the chip;
3. Passing the test antigen over the chip's capture surface at 1024nM, 256nM, 64nM, 16nM, 4nM with a 0nM (i.e. buffer alone); and
4. And determining the affinity of binding of test antibody/chain to test antigen using surface plasmon resonance, eg, under an SPR condition discussed above (eg, at 25oC in physiological buffer). SPR can be carried out using any standard SPR apparatus, such as by BiacoreTM or using the ProteOn XPR36TM (Bio-Rad^{®}).

Regeneration of the capture surface can be carried out with 10mM glycine at pH1.7. This removes the captured antibody and allows the surface to be used for another interaction. The binding data can be fitted to 1:1 model inherent using standard techniques, eg, using a model inherent to the ProteOn XPR36TM analysis software.

The term "antibody" includes monoclonal antibodies (including full length antibodies which have an immunoglobulin Fc region), antibody compositions with polyepitopic specificity, multispecific antibodies (e.g., bispecific antibodies, diabodies, and single-chain molecules, as well as antibody fragments (e.g., Fab, F(ab')2) having paired VH/VL antigen binding site(s).

A "domain" is a folded protein structure which has tertiary structure independent of the rest of the protein. Generally, domains are responsible for discrete functional properties of proteins and in many cases may be added, removed or transferred to other proteins without loss of function of the remainder of the protein and/or of the domain.

Optionally, any multispecific antibody herein is a bispecific antibody. This includes formats such as DVD-Ig, mAb², FIT-Ig, mAb-dAb, dock and lock, Fab-arm exchange, SEEDbody, Triomab, LUZ-Y, Fcab, κλ-body, orthogonal Fab, scDiabody-Fc, diabody-Fc, tandem scFv-Fc, Fab-scFv-Fc, Fab-scFv, intrabody, BiTE, diabody, DART, TandAb, scDiabody, scDiabody-CH3, Diabody-CH3, Triple body, Miniantibody, minibody, TriBi minibody, scFv-CH3 KIH, scFv-CH-CL-scFv, F(ab')2-scFv, scFv-KIH, Fab-scFv-Fc, tetravalent HCab, ImmTAC, knobs-in-holes, knobs-in-holes with common light chain, knobs-in-holes with common light chain and charge pairs, charge pairs, charge pairs with common light chain, DT-IgG, DutaMab, IgG(H)-scFv, scFv-(H)IgG, IgG(L)-scFv, scFv-(L)IgG, IgG(L,H)-Fv, IgG(H)-V, V(H)-lgG, IgG(L)-V, V(L)-IgG, KIH IgG-scFab, 2scFv-lgG, IgG-2scFv, scFv4-lg and zybody. For a review of bispecific formats, see Spiess, C., et al.,. Mol. Immunol. (2015). Optionally, the multispecific ligand is a DVD-Ig, mAb², FIT-Ig, mAb-dAb, dock and lock, SEEDbody, scDiabody-Fc, diabody-Fc, tandem scFv-Fc, Fab-scFv-Fc, Fab-scFv, intrabody, BiTE, diabody, DART, TandAb, scDiabody, scDiabody-CH₃, Diabody-CH₃, minibody, knobs-in-holes ligand, knobs-in-holes ligand with common light chain, knobs-in-holes ligand with common light chain and charge pairs, charge pairs, ligand having charge pairs with common light chain; in each case comprising at least one VH/VL antigen binding site.

Optionally, the rearranged variable region (eg, a rearranged variable region encoding a VL, Vλ or Vκ domain, eg, a human domain) in the light chain locus comprises upstream one of the following pairs of promoter and signal peptide (ie, promoter, SP and V region in 5' to 3' order):-
1) Mouse Vλ promoter + Mouse Vλ SP
2) Mouse Vλ promoter + Non-mouse rodent Vλ SP
3) Mouse Vλ promoter + Human Vλ SP
4) Mouse Vλ promoter + Non-human primate Vλ SP
5) Mouse VK promoter + Mouse Vλ SP
6) Mouse VK promoter + Non-mouse rodent Vλ SP
7) Mouse VK promoter + Human Vλ SP
8) Mouse VK promoter + Non-human primate Vλ SP
9) Non-mouse rodent Vλ promoter + Mouse Vλ SP
10) Non-mouse rodent Vλ promoter + Non-mouse rodent Vλ SP
11) Non-mouse rodent Vλ promoter + Human Vλ SP
12) Non-mouse rodent Vλ promoter + Non-human primate Vλ SP
13) Non-mouse rodent VK promoter + Mouse Vλ SP
14) Non-mouse rodent VK promoter + Non-mouse rodent Vλ SP
15) Non-mouse rodent VK promoter + Human Vλ SP
16) Non-mouse rodent VK promoter + Non-human primate Vλ SP
17) Human Vλ promoter + Mouse Vλ SP
18) Human Vλ promoter + Non-mouse rodent Vλ SP
19) Human Vλ promoter + Human Vλ SP
20) Human Vλ promoter + Non-human primate Vλ SP
21) Human VK promoter + Mouse Vλ SP
22) Human VK promoter + non-mouse rodent Vλ SP
23) Human VK promoter + Human Vλ SP
24) Human VK promoter + Non-human primate Vλ SP
25) Non-human primate Vλ promoter + Mouse Vλ SP
26) Non-human primate Vλ promoter + Non-mouse rodent Vλ SP
27) Non-human primate Vλ promoter + Human Vλ SP
28) Non-human primate Vλ promoter + Non-human primate Vλ SP
29) Non-human primate VK promoter + Mouse Vλ SP
30) Non-human primate VK promoter + Non-mouse rodent Vλ SP
31) Non-human primate VK promoter + Human Vλ SP
32) Non-human primate VK promoter + Non-human primate Vλ SP
33) Mouse Vλ promoter + Mouse VK SP
34) Mouse Vλ promoter + Non-mouse rodent VK SP
35) Mouse Vλ promoter + Human VK SP
36) Mouse Vλ promoter + Non-human primate VK SP
37) Mouse VK promoter + Mouse VK SP
38) Mouse VK promoter + Non-mouse rodent VK SP
39) Mouse VK promoter + Human VK SP
40) Mouse VK promoter + Non-human primate VK SP
41) Non-mouse rodent Vλ promoter + Mouse VK SP
42) Non-mouse rodent Vλ promoter + Non-mouse rodent VK SP
43) Non-mouse rodent Vλ promoter + Human VK SP
44) Non-mouse rodent Vλ promoter + Non-human primate VK SP
45) Non-mouse rodent VK promoter + Mouse VK SP
46) Non-mouse rodent VK promoter + Non-mouse rodent VK SP
47) Non-mouse rodent VK promoter + Human VK SP
48) Non-mouse rodent VK promoter + Non-human primate VK SP
49) Human Vλ promoter + Mouse VK SP
50) Human Vλ promoter + Non-mouse rodent VK SP
51) Human Vλ promoter + Human VK SP
52) Human Vλ promoter + Non-human primate VK SP
53) Human VK promoter + Mouse VK SP
54) Human VK promoter + non-mouse rodent VK SP
55) Human VK promoter + Human VK SP
56) Human VK promoter + Non-human primate VK SP
57) Non-human primate Vλ promoter + Mouse VK SP
58) Non-human primate Vλ promoter + Non-mouse rodent VK SP
59) Non-human primate Vλ promoter + Human VK SP
60) Non-human primate Vλ promoter + Non-human primate VK SP
61) Non-human primate VK promoter + Mouse VK SP
62) Non-human primate VK promoter + Non-mouse rodent VK SP
63) Non-human primate VK promoter + Human VK SP
64) Non-human primate VK promoter + Non-human primate VK SP

Example rodents are rat, beaver, gopher, hamster, gerbil, porcupine, chinchilla, lemming or vole. Example non-human primates are chimpanzee, ape, gorilla, orangutan, simian, monkey (eg, rhesus macaque or cynonmolgus monkey), lemur or baboon. Sources of sequences for the promoter and/or SP include, for example, ww.imgt.org, www.ensembl.org and sequences obtained using a sample (eg, blood sample and PCR of the sequence) from such a rodent or primate or human, as will be readily apparent to the skilled addressee.

In an example, the light chain locus of the cell, vertebrate, mammal or mouse of the invention is devoid of a selection marker. In the exaples below, a selection marker was retained and the locus performed desirably. In an alternative, an excisionable selection marker can be included when constructing the locus, followed by excsission of the marker before developing the vertebrate or mouse. In an example the marker is flanked by piggyBac terminal repeats and PBase is transiently expressed in the ES cell in which the locus has been constructed, thereby excising the marker. In an example, the marker compriss puro-delta-tk flanked by piggyBac terminal repeats.

The disclosure further provides a composition (eg, a pharmaceutical composition or a composition for medical use) comprising an antibody, bispecific antibody, antibody chain (eg, light chain), VL or nucleotide sequence thereof obtained or obtainable by a method of the invention as disclosed herein; optionally wherein the composition comprises a diluent, excipient or carrier, optionally wherein the composition is contained in an IV container (eg, and IV bag) or a container connected to an IV syringe. When the composition is a pharmaceutical composition or a composition for medical use, the diluent, excipient or carrier is pharmaceutically acceptable. "Pharmaceutically acceptable" refers to approved or approvable by a regulatory agency of the USA Federal or a state government or listed in the U.S. Pharmacopeia or other generally recognized pharmacopeia for use in animals, including humans. A "pharmaceutically acceptable carrier, excipient, or adjuvant" refers to a carrier, excipient, or adjuvant that can be administered to a subject, together with an agent, e.g., any antibody, VL or antibody chain described herein, and which does not destroy the pharmacological activity thereof and is nontoxic when administered in doses sufficient to deliver a therapeutic amount of the agent.

The singular terms "a," "an," and "the" include plural referents unless context clearly indicates otherwise. Similarly, the word "or" is intended to include "and" unless the context clearly indicates otherwise. Although methods and materials similar or equivalent to those described herein can be used in the practice or testing of this disclosure, suitable methods and materials are described below. The abbreviation, "e.g." is derived from the Latin exempli gratia, and is used herein to indicate a non-limiting example. Thus, the abbreviation "e.g." is synonymous with the term "for example."

Definitions of common terms in cell biology and molecular biology can be found in "The Merck Manual of Diagnosis and Therapy", 19th Edition, published by Merck Research Laboratories, 2006 (ISBN 0-911910-19-0); Robert S. Porter et al. (eds.), The Encyclopedia of Molecular Biology, published by Blackwell Science Ltd., 1994 (ISBN 0-632-02182-9); Benjamin Lewin, Genes X, published by Jones & Bartlett Publishing, 2009 (ISBN-10: 0763766321); Kendrew et al. (eds.), , Molecular Biology and Biotechnology: a Comprehensive Desk Reference, published by VCH Publishers, Inc., 1995 (ISBN 1-56081-569-8) and Current Protocols in Protein Sciences 2009, Wiley Intersciences, Coligan et al., eds.

Unless otherwise stated, the present invention was performed using standard procedures, as described, for example in Sambrook et al., Molecular Cloning: A Laboratory Manual (4 ed.), Cold Spring Harbor Laboratory Press, Cold Spring Harbor, N.Y., USA (2012); Davis et al., Basic Methods in Molecular Biology, Elsevier Science Publishing, Inc., New York, USA (1995); or Methods in Enzymology: Guide to Molecular Cloning Techniques Vol.152, S. L. Berger and A. R. Kimmel Eds., Academic Press Inc., San Diego, USA (1987); Current Protocols in Protein Science (CPPS) (John E. Coligan, et. al., ed., John Wiley and Sons, Inc.), Current Protocols in Cell Biology (CPCB) (Juan S. Bonifacino et. al. ed., John Wiley and Sons, Inc.), and Culture of Animal Cells: A Manual of Basic Technique by R. Ian Freshney, Publisher: Wiley-Liss; 5th edition (2005), Animal Cell Culture Methods (Methods in Cell Biology, Vol. 57, Jennie P. Mather and David Barnes editors, Academic Press, 1st edition, 1998).

Other terms are defined herein within the description of the various aspects of the invention.

All patents and other publications; including literature references, issued patents, published patent applications, and co-pending patent applications (including the US counterparts of any of these); cited throughout this application are provided solely for their disclosure prior to the filing date of the present application. Nothing in this regard should be construed as an admission that the inventors are not entitled to antedate such disclosure by virtue of prior invention or for any other reason. All statements as to the date or representation as to the contents of these documents is based on the information available to the applicants and does not constitute any admission as to the correctness of the dates or contents of these documents.

The description of embodiments of the disclosure is not intended to be exhaustive or to limit the disclosure to the precise form disclosed. While specific embodiments of, and examples for, the disclosure are described herein for illustrative purposes, various equivalent modifications are possible within the scope of the disclosure, as those skilled in the relevant art will recognize. For example, while method steps or functions are presented in a given order, alternative embodiments may perform functions in a different order, or functions may be performed substantially concurrently. The teachings of the disclosure provided herein can be applied to other procedures or methods as appropriate. The various embodiments described herein can be combined to provide further embodiments. Aspects of the disclosure can be modified, if necessary, to employ the compositions, functions and concepts of the above references and application to provide yet further embodiments of the disclosure. Moreover, due to biological functional equivalency considerations, some changes can be made in protein structure without affecting the biological or chemical action in kind or amount. These and other changes can be made to the disclosure in light of the detailed description. All such modifications are intended to be included within the scope of the appended claims.

Specific elements of any of the foregoing embodiments can be combined or substituted for elements in other embodiments. Furthermore, while advantages associated with certain embodiments of the disclosure have been described in the context of these embodiments, other embodiments may also exhibit such advantages, and not all embodiments need necessarily exhibit such advantages to fall within the scope of the disclosure.

It will be understood that particular configurations, aspects, examples, embodiments described herein are shown by way of illustration and not as limitations of the invention. The principal features of this invention can be employed in various embodiments without departing from the scope of the invention. Those skilled in the art will recognize, or be able to ascertain using no more than routine study, numerous equivalents to the specific procedures described herein. Such equivalents are considered to be within the scope of this invention and are covered by the claims. All publications and patent applications mentioned in the specification are indicative of the level of skill of those skilled in the art to which this invention pertains. The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the device, the method being employed to determine the value, or the variation that exists among the study subjects.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

Any part of this disclosure may be read in combination with any other part of the disclosure, unless otherwise apparent from the context.

All of the compositions and/or methods disclosed and claimed herein can be made and executed without undue experimentation in light of the present disclosure. While the compositions and methods of this invention have been described in terms of preferred embodiments, it will be apparent to those of skill in the art that variations may be applied to the compositions and/or methods and in the steps or in the sequence of steps of the method described herein without departing from the concept, scope of the invention. All such similar substitutes and modifications apparent to those skilled in the art are deemed to be within the scope and concept of the invention as defined by the appended claims.

The present invention is described in more detail in the following non limiting Examples.

### EXAMPLES

### Example 1. Construction of targeting vectors for mouse ES cell targeting

### 1.1 pUC57_N128L_KI vector DNA synthesis

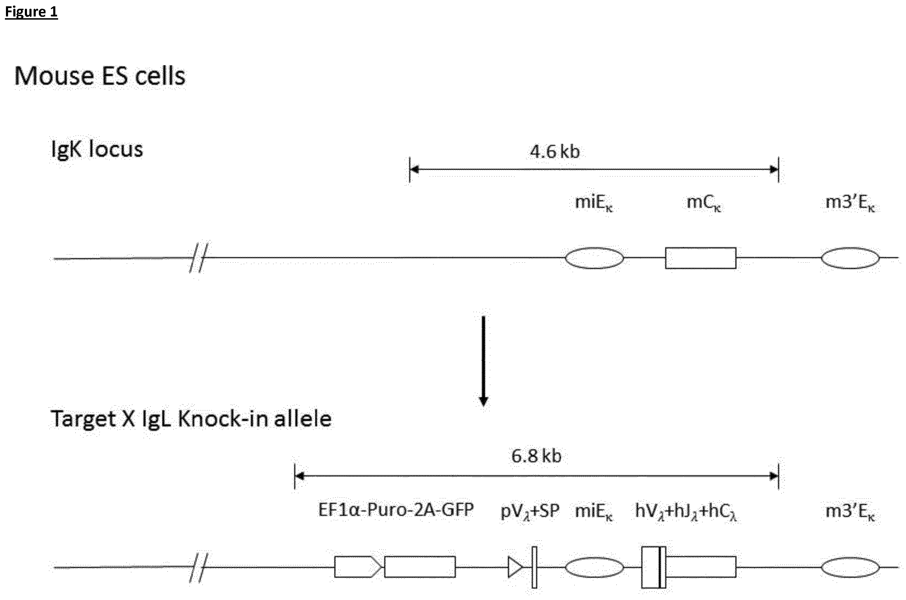

N128L is a human λ light chain rearranged from human IGLV3-21*01 and IGU3*02 (VL domain sequence is given below). A DNA fragment containing wildtype human IGLV3-21*01 promoter (pV_{λ}) and signal peptide (SP), mouse intronic κ enhancer (miE_{κ}), and N128L light chain variable region and constant region was synthesized by Genscript. A 1-kb fragment between V_{κ} and J_{κ} region in the kappa locus, and a 1-kb fragment between mouse κ constant region (C_{κ}) and mouse 3' κ enhancer (m3'E_{κ}) were used as homology arms for targeting the N128L knock-in cassette. After successful targeting, kappa allele DNA including mouse C_{κ} was replaced by the N128L knock-in (Figure 1).

### 1.2 pUC57_N128L_EF1α_Puro_2A_EGFP_SV40pA targeting vector

EF1α_Puro_2A_EGFP_SV40pA vector was digested with Notl and Ascl. A 3.6 kb fragment was purified using QIAquick^{™} PCR Purification Ki (QIAGEN) by the method described in the attached instruction manual. pUC57_N128L_KI vector was digested with Notl and Ascl. A 7.9 kb fragment was purified QIAquick PCR Purification Kit (QIAGEN) by the method described in the attached instruction manual. The Notl-Ascl-digested EF1α_Puro_2A_EGFP_SV40pA and pUC57_N128L_KI fragments were ligated using T4 ligase (New England Biolabs) according to the method described in the attached instruction manual. *E.coli* DH10B strain (ElectroMax^{™} DH10B (Invitrogen)) was transformed with the ligation solution. Respective plasmid DNAs were isolated from the obtained ampicillin resistant clones using QIAprep^{™} Spin Miniprep Kit (QIAGEN). The resulting respective ampicillin resistant transformants were confirmed to have the insertion by Sanger sequencing.

### 1.3 Preparation of plasmid DNA for mouse ES cell targeting

For pUC57_N128L_EF1α_Puro_2A_EGFP_SV40pA targeting vector, plasmid DNA was isolated from the sequencing verified clones using QlAprep Maxi plus Kit^{™} (QIAGEN) by the method described in the attached instruction manual. Respective plasmid DNAs were confirmed to have the insertion by Sanger sequencing.

### Example 2. Generation of N128L knock-in mouse ES cell lines

### 2.1 Preparation of mouse ES cells

Cells from two independent mouse embryonic stem cell (ES) lines were expanded on STO feeder plates for electroporation. ES cells were fed with fresh M15 media until they reached 80% to 85% confluence under microscope. The knock-in construct was introduced using electroporation.

ES cells were cultured in M15 media supplemented with Puromycin (1 µg/mL). Seven days after electroporation, Puromycin resistant colonies were big enough for picking.

### 2.2 Microinjection of targeted mouse ES cell clones

Targeted ES cells were injected to C57BL6 strain blastocysts. After injection, blastocysts were transferred to the uterus of C57BL6 strain F1 hybrid females mated with vasectomised males 3 days before injection. The chimaeric embryos were allowed to develop to term in the pseudo-pregnant recipients, which developed into pups comprising the light chain knock-in.

### Example 3: Common Light Chain Mice

Eight chimaera mice with common light chain knock-in allele and a functional unrearranged human VH region in an IgH locus were immunized with human Target X.

The knock-in encodes for the following N128L VL (in N- to C-terminal direction):-

SYVLTQPPSVSVAPGETARITCGGDNIGRKSVYWYQQKSGQAPVLVIYYDSDRPSGIPERFSGSNSGNTATLTISRVE
AGDEADYYCQVWDGSSDHWVFGGGTKLTVL

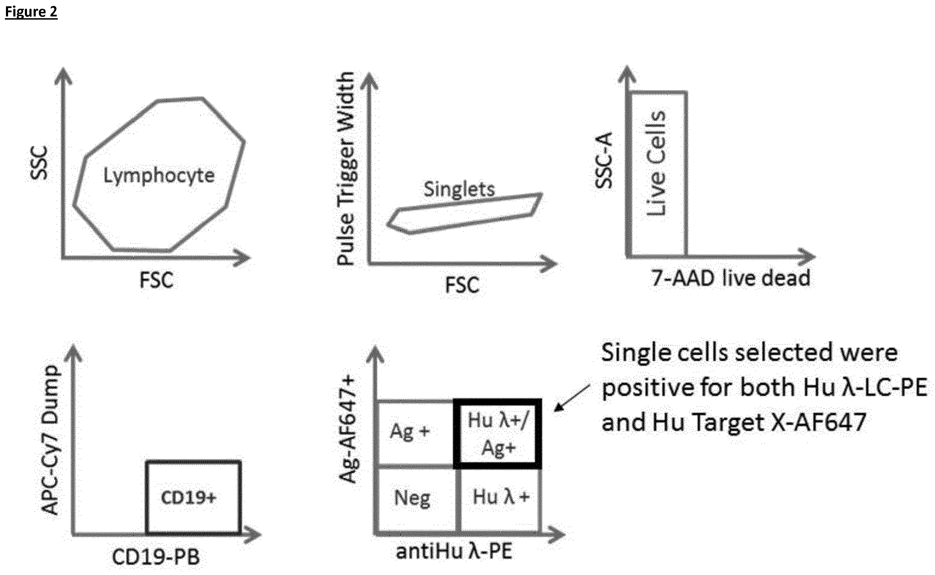

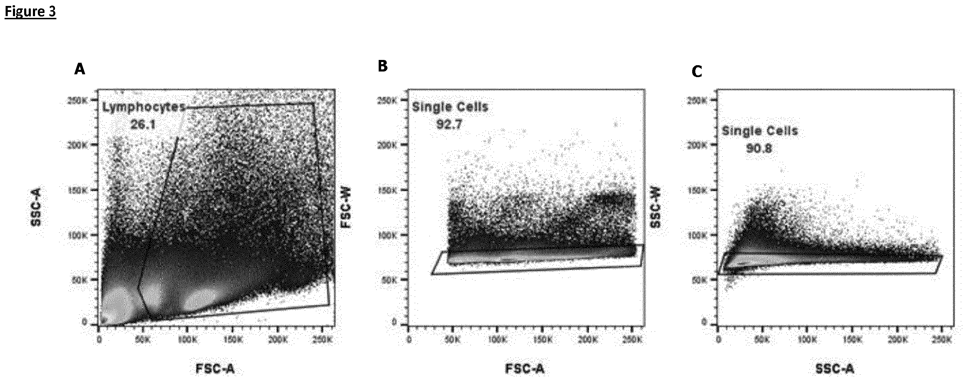

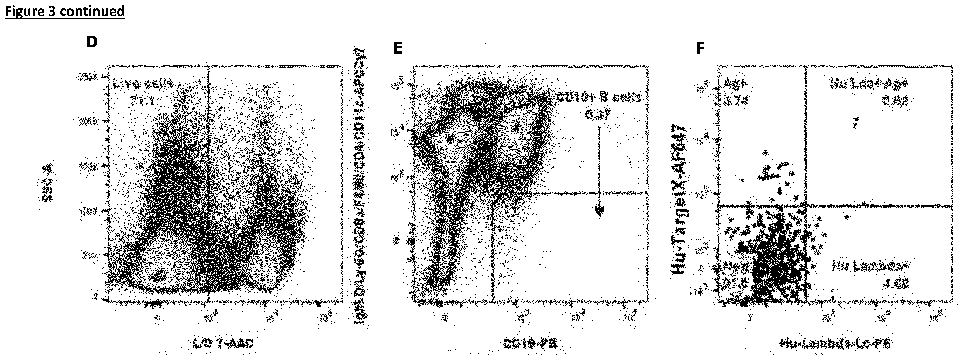

After immunization, sorting was performed on spleen B cells using a monoclonal antibody against human λ light chain labelled with PE and human Target X labelled with AF647 (Figs 2 & 3). B cells that were positive for both PE and AF647 were sorted as single cell into individual wells on a 96-well plate. Single cell NGS libraries were constructed using standard protocol and subjected to NGS sequencing.

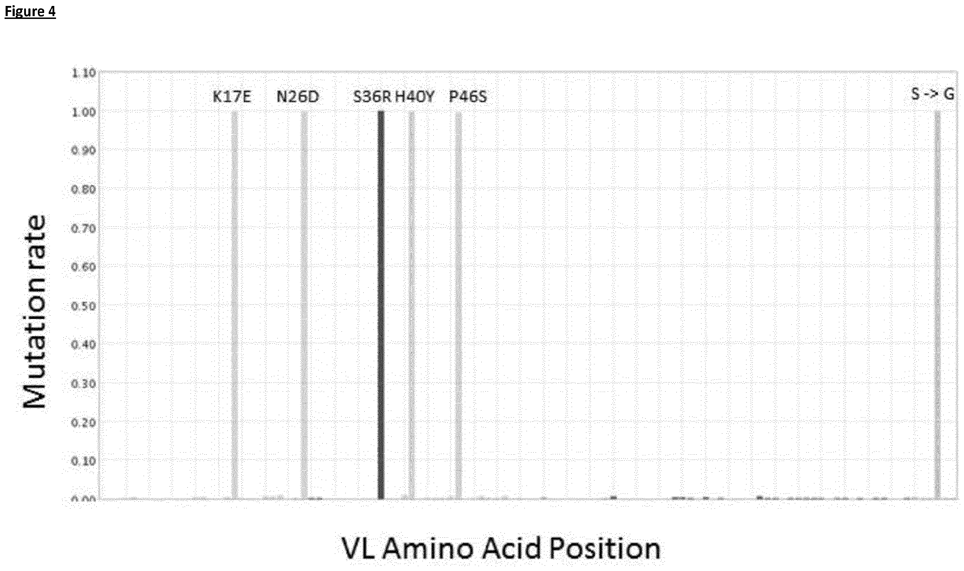

After subtraction of sequences from a PCR control included in our experiment, we found 346 clones, all of which comprised the expected λ light chain combination derived from IGLV3-21*01 and IGLJ3*02. Clustering the light chain sequences with that of the knock-in light chain allele demonstrated that 339 out of 346 clones were unmutated, with the other 7 clones having one or more mutations (Fig 4).

**Table 1: Common L Chain Frequencies**

| **Number of Clones (out of 346 total)** | **Percent Identity to predetermined human λ light chain rearranged from human IGLV3-21*01 and IGLJ3*02** |
|---|---|
| 339 | 100.00% |
| 1 | 97.23% |
| 1 | 98.15% |
| 1 | 99.08% |
| 4 | 99.69% |

Compared to sequences that are a perfect (ie, 100%) match for the inserted light chain sequence, ones that do not completely match have lower confidence scores (81.6% confidence score (for the average of the sequences assigned a 100% match) versus 31.7% confidence score (for the average of the 7 other sequences), 100% represents the highest confidence, 0% represents the lowest confidence). Thus, we found that of all the VLs that were based on the human IGLV3-21*01 and IGLJ3*02 combination, 98% comprised the identical input common light chain V sequence.

After bioinformatics analysis, desirably the human heavy chain V domain sequence repertoire of the clones was found to be diverse. There were no dominant human V_{H}, D_{H} or J_{H} usage identified by the bioinformatics analysis. This matches the bulk NGS analysis of variable regions of heavy chains in spleen B cells from naive chimera mice (data not shown).

Human VH gene segment usage comprised the following:
IGHV1-3*01, IGHV1-8*01, IGHV1-18*01, IGHV1-46*03, IGHV3-7*01, IGHV3-9*01, IGHV3-11*01, IGHV3-15*01, IGHV3-20*d01, IGHV3-21*03, IGHV3-23*04, IGHV3-30*18, IGHV3-33*01, IGHV3-48*02, IGHV3-53*01, IGHV4-4*02, IGHV4-31*03, IGHV4-34*01, IGHV4-39*01, IGHV4-59*01, IGHV4-61*01, IGHV5-51*01, IGHV6-1*01 and IGHV7-4-1*01.

### Example 4: Construction of Common Light Chain Targeting Vector For Mouse ES Cell Targeting

Further common light chain mice were made as follows.

### Generation of common light chain targeting vector

A DNA fragment containing wild-type human V_{λ} promoter (pV_{λ}) and signal peptide (SP), mouse intronic κ enhancer (miE_{κ}), and nucleotide sequence encoding a human light chain variable region and constant region was synthesized. The light chain variable domain has specificity for a target (Target Z) other than Target Y used below. A 1kb fragment between V_{κ} and J_{κ} (1-5), and a 1kb fragment between mouse κ constant region (C_{κ}) and mouse 3' κ enhancer (m3'E_{κ}) were used as homology arms for targeting the common light chain knock-in cassette. After successful targeting, the human J_{κ} (1-5) and mouse C_{κ} will be replaced by common light chain knock-in cassette on one or both alleles.

### Generation of common light chain targeted mouse ES cells

Cells from two independent mouse embryonic stem cell (ES) lines were expanded on STO feeder plates for electroporation. ES cells were fed with fresh M15 media until they reached 80% to 85% confluence under microscope.

Two hours before electroporation, ES cells were fed with fresh M15 media. Before electroporation, ES cells were washed twice with PBS, and dissociated from plates by adding trypsin. After incubation at 37°C for 20 minutes, the trypsin was inactivated by adding same volume of M15 media. ES cells were dissociated to a single cell suspension by repeat pipetting. ES cells were pelleted by centrifuging at 1,100 rpm for 4 minutes, and re-suspended in electroporation buffer (Lonza) by the method described in the attached instruction manual.

Electroporation were performed using Lonza Amaxa^{™} machine using preset mouse ES cell protocol. After electroporation, ES cells from each electroporation were re-suspended in fresh M15 media, and plated onto one 10 cm feeder plate.

ES cells were fed with M15 media for 3 days after electroporation. ES cells from each electroporation were passaged 1:3 to three 10 cm feeder plates. Two days after passage, ES cells were cultured in M15 media supplemented with Puromycin (1 µg/mL). Seven days after electroporation, Puromycin resistant colonies were big enough for picking.

Puromycin resistant colonies on 10 cm STO feeder plates were fed with fresh M15 media 2 hours before picking. 50 µl of trypsin was added to each well of a 96-well round bottom plate. Colonies on 10 cm STO feeder plates twice with PBS buffer. 10 ml of PBS buffer was added to each 10cm plate.

Single colonies were picked from the feeder plates using a Gilson pipette, and transferred into the well with trypsin of 96-well plate (1 colony/well). After completing a plate (96 colonies picked), the trypsin plate was incubated at 37°C for 30 minutes. After that, 150 µl of fresh M15 media was added to each well. Colonies were dissociated by repeat pipetting. Single cell suspension was transferred to a 96-well feeder plate and cultured in a TC incubator at 37°C. ES cells were fed with fresh M15 media until they reached 80% to 85% confluence under microscope.

### Passaging common light chain mouse ES cells

Two hours before passaging, ES cells were fed with fresh M15 media. Before passaging, ES cells were washed twice with PBS buffer, and dissociated from plates by adding trypsin. After incubation at 37°C for 20 minutes, the trypsin was inactivated by adding same volume of M15 media. ES cells were dissociated to a single cell suspension by repeat pipetting.

Single ES cell suspension were split 1:4 to four 96 well STO feeder plates. ES cells were fed with fresh M15 media until they reached 80% to 85% confluence under microscope.

### Freezing ES cells on 96 well plate

Two hours before freezing, ES cells were fed with fresh M15 media. Before freezing, ES cells were washed twice with PBS buffer, and dissociated from plates by adding trypsin. After incubation at 37°C for 20 minutes, the trypsin was inactivated by adding same volume of 2X freezing media (60% DMEM, 20% FBS, 20% DMSO, freshly prepared). ES cells were dissociated to a single cell suspension by repeat pipetting. 96 well plates were sealed and placed in a polystyrene box inside a -80°C freezer to facilitate a very slow freezing process.

### Genomic DNA extraction

ES cells were washed twice with PBS buffer, and 50 µL of ES cell Lysis Buffer (10 mM Tris-HCl, pH 7.5, 10 mM EDTA, 0.5% SDS, 10 mM NaCl) with Proteinase K (1 mg/mL) was added to each well of the 96 well plates. The plates were sealed, wrapped with wet tissues, and placed into a lunch box. The lunch box was incubated at 55 °C overnight.

The second day, 100 µL ice-cold Ethanol/NaCl mix (1.5 mL 5M NaCl added to 98.5 mL 100% ethanol) was added to each well of the 96 well plates. The plates were at room temperature for about 30 minutes. After incubation, the plates were centrifuged at 3,000 rpm for 20 minutes. The plates were inverted to decant liquid, and dried on paper towels to remove access liquid. The plates were washed twice by adding 150 µL cold 70% ethanol, and dried completely on paper towels. After the DNA pellets were dried, 30 µL of TE (pH 8.0) buffer was added and DNA was resuspended by incubation at 55 °C for 30 minutes.

### Thawing and expansion of common light chain mouse ES cell clones

Frozen 96 well plates were taken out of the -80°C freezer, and thawed in a 37°C incubator until every well of the plates became transparent. 2 mL fresh M15 media was added to each well of 24 well STO feeder plates. All the contents from the desired wells of the 96 well plates were transferred to a well of the 24 well plates. After all the targeted clones were transferred to a 24 well plate, the plate was cultured in a TC incubator at 37°C. ES cells were fed with fresh M15 media until they reached 80% to 85% confluence under microscope. Once they became confluent, they were 1:1 passaged to 6-well STO feeder plates and frozen down for microinjection.

### Microinjection of common light chain mouse ES cell clones

Mouse ES cells were thawed and expanded before injection. Two hours before injection, ES cells were fed with fresh M15 media. Before injection, ES cells were washed twice with PBS buffer, and dissociated from plates by adding trypsin. After incubation at 37°C for 20 minutes, the trypsin was inactivated by adding same volume of fresh M15 media. ES cells were dissociated to a single cell suspension by repeat pipetting, and put on ice for injection.

Targeted ES cells were injected to E3.5 Rag^{-/-} blastocysts. After injection, blastocysts were transferred to the uterus of CBA;C57BL6/J F1 hybrid females mated with vasectomised males 3 days before injection. The chimeric embryos were allowed to develop to term in the pseudo-pregnant recipients. The resulting mice comprised cells derived from the engineered ES cells, where the cell genomes comprised the human lambda light chain knock-in at one mouse kappa locus (the other kappa locus was inactivated by deletion in the J region) and an unrearranged human heavy chain variable region comprised by a homozygous IgH locus.

### Example 5: Production of Antibody To Target Y

### Immunization

Common light chain chimera mice were immunized with Target Y as described below. As the first immunization, 40 µg/head of Target Y, emulsified by CpG (Sigma) and Alum (1:1 antigen), was subcutaneously administered. Four weeks later, 5 µg/head of Target Y, emulsified by by CpG (Sigma) and Alum (1:1 antigen) was administered by intraperitoneal injection (first boost). One week later, four mice were sacrificed and the spleens were extracted. Four weeks after the first boost, 2 µg/head of Target Y, emulsified by by CpG (Sigma) and Alum (1:1 antigen) was administered by intraperitoneal injection (second boost) to the another four mice One week later these mice were sacrificed, and the spleens were extracted.

### Antigen specific B cell sorting

Harvested and processed spleen tissues were resuspended in 20 mL 3% FBS/PBS. Cells were pelleted at 300 *g* for 10 seconds at 40°C, and resuspended in 300 µl 3% FBS/PBS buffer.

20ul of Fc blocker was added for 10 seconds and stored at 4°C before use.

Alexa-647 labelled human Target Y stock solution (0.8 mg/mL) was diluted to 20 µg/mL and used at 1:40 dilution (final concentration 0.5 µg/mL) for staining.

A 100 µl staining cocktail containing CD19-PB, IgM-APC-Cy7, IgD-APC-Cy7, CD38-FITC, CD95-PE, Hu-Target Y - Alexa 647, was mixed and added to 300 µl spleen cell suspension. After incubation at 4°C for 20 minutes, 5 ml of 3% FBS/PBS buffer was added to the samples. Cells were pelleted at 300 *g* for 10 minutes, and resuspended in 400 µl of 3% FBS/PBS buffer and filter through 30 µm cell strainer. Five minutes before sorting, 8 µl of 7AAD (1:100 dilution) was added. The gating strategy for sorting is shown below.

### B-Cell Screening (BCT) and recovery of coupled antibody heavy and light chains for expression

BCT technology is generally described in WO2015/040401.

For RT, a mixture of 0.1 µL of a IgG constant region specific primer (10 µM), 0.1 µL of a IgK constant region specific primer (10 µM), 0.05 µL a IgL constant region specific primer (10 µM), 1 µL dNTP mix (10 mM), 2 µL of 5X First-Strand Buffer, 1 µL of 0.1 M DTT, 0.4 µL of RNaseOUT^{™} RNase Inhibitor (Invitrogen), 0.25 µL of Superscript^{™} III RT (200 units/µl, Invitrogen), and 1.1 µL of RNase-free water were added to each well to make a final volume of 10 µL. RT reactions were performed to generate 1^{st} strand cDNA.

For 1^{st} round PCR, a mixture of 12.5 µL of 2X Q5 master mix, 0.15 µL of a mixture of VH specific primers (10 µM), 0.1 µM of a mixture of VL specific primers (10 µM), 0.15 µL of a mixture of VK specific primers (10 µM), 0.1 µL of a IgG constant region specific primer (10 µM), 0.1 µL of a IgK constant region specific primer (10 µm), 0.1 µL of a IgL constant region specific primer (10µm), and 1.8 µL of RNase-free water was mixed with 10 µL of RT product in each well to make a final volume of 25 µL. PCR reactions were performed to amplify heavy chain and light chain variable regions.

For 2^{nd} round PCR, a mixture of 12.5 µL of 2X Q5 master mix, 0.2 µL of a VH nested PCR primer (10 µM), 0.2 µL of a VK nested PCR primer (10 µM), 0.2 µL of a VL nested PCR primer (10 µM), 0.2 µL of a IgG constant region nested PCR primer (10 µM), 0.2 µL of a IgK constant region nested PCR primer (10 µM), 0.2 µL of a IgL constant region nested PCR primer (10 µM), and 10.3 µL of RNase-free water was mixed with 1 µL of 1^{st} round PCR product in each well to make a final volume of 25 µL. PCR reactions were performed to amplify heavy chain and light chain variable regions.

For bridge PCR, a mixture of 15 µL of 2X Q5 master mix, 0.1 µL of Heavy Vector (100 ng/ µl), 0.05 µL of Kappa vector (100ng/µl), and 1 µL of HLP479/480 mix (10 µM each), and 12.85 µL of RNase-free water was mixed with 1 µL of 2^{nd} round PCR product in each well to make a final volume of 25 µL. PCR reactions were performed to amplify heavy chain and light chain variable regions.

### Expi293F cell transfection

See Examples 6 and 7 for more details of antibody expression and purification.

One day before transfection, cultured Expi293F cells (Invitrogen) were counted for cell seeding calculation. Expi293F cells were pelleted at 300 rpm for 10 minutes. Cells were resuspended in pre-warmed fresh Expi293 expression media to give a final dilution of 2.5×10⁶ cells/ml. 200 ml of cell suspension was incubated in a Kuhner Shaking Incubator (37°C, 140 rpm, 8% CO₂) until next morning.

On the day of transfection, cultured Expi293F cells were counted and diluted to 4×10⁶ cells/ml using fresh Expi293 expression media. 500 µl of cell suspension was aliquoted into each well of 96-well deep well plates using Multidrop Combi. After dispensing, the plates were covered with Duetz sandwich cover, and incubated in a Kuhner Shaking Incubator (37°C, 300 rpm, 50 mm orbital throw, humidity 80%) for 2-2.5 hours.

For each transfection, two mixtures were prepared.
Mix 1: 25µl B cell bridge product + 55µl RSM + 100ng PBase per well
Mix 2: 1µl ExpiFectamine^{™} 293 (Invitrogen) + 79µl RSM

Mix 2 was added to Mix 1 and incubated at room temperature for 15 minutes. The incubated mixture was then added to the 500 µl of cell culture solution and incubated in a Kuhner Shaking Incubator (37°C, 300 rpm, 50mm orbital throw) for a week.

### Example 6: Expression of Antibodies

Mammalian transient expression systems enable flexible and rapid production of proteins. They are ideal for expression of human or other mammalian proteins because these systems generate recombinant proteins with more native folding and post-translational modifications.
1. The day before transfection, Expi293 cells were counted by an automated cell counter (Eve cell counter) seeded in pre-warmed expression media at the density of ~1.7×10⁶cells/ml and incubated overnight in an orbital shaker incubator (37°C, 8% CO2, 140 rpm)
2. On the day of transfection, cells were counted and adjusted the cell number to 2.5×10⁶cells/ml. 2000µl of cell suspension were dispensed into 24 Deep Well Plates and placed into Kuhner shaking incubator (37°C, 8% CO2, 225rpm)

### Preparation of DNA solutions

Mixed solutions containing plasmid DNA were prepared for transfection of Expi293 cells. For 2 mL of cell culture, 1 µg each ofthe heavy chain expression plasmid and the light chain expression plasmid were used. 2µg of DNA plasmid mixture diluted in ultrapure water was used for each transfection.

### Transfection

Two mixtures were prepared:
Mix 1: 2µg of plasmid DNA diluted in 40µl of Opti-MEM^{®} I medium.
Mix 2: 80µl of ExpiFectamine^{™} 293 transfection reagent + 40µl of OptiMEM I medium.

Mix 2 was combined with Mix 1 and incubated for 20-30 minutes at room temperature. After the incubation was complete 165µl of DNA-ExpiFectamine^{™} 293 Reagent complex was dispensed to each well of the 24 deep well plate. Cells were incubated in the Kuhner shaking incubator (37°C, 8% CO2, 225rpm) for 6 days. Cell culture supernatants were harvested 6 days after transfection.

### Example 7: Purification of Antibodies from Common Light Chain Mouse

A 96-well plate purification was employed to purify antibodies from Expi293F cell culture supernatants. This method allows rapid small-scale affinity antibody purification of multiple samples in antibody screening experiments. To achieve high % of recovery MabSelect Sure LX^{™} was used which is a protein A affinity with very dynamic binding activity (60 mg human IgG/ml medium), an extended residence time, alkali tolerant and low ligand leakage.

### Procedure

1. MabSelect Sure LX resin (GE Healthcare) was equilibrated in 1X PBS (Gibco) to remove the storage buffer, to a final concentration of 10 % slurry.
2. 600 µl of 10 % slurry is added to each well of the 96 well plate AcroPrep^{™} Advance (Pall)
3. The resin was centrifuged 70 × rcf for 1 min at 4 C.
4. The resin was washed with 300 µl 1X PBS, spin 70x rcf for 1 min. This step was repeated twice.
5. 2 ml of cell culture supernatants (pH 7-8) were loaded onto the MabSelect Sure LX resin into the 96 well purification plate and centrifuged at 70 - 100 × rcf for 1 minute.
6. Plate was washed using 600 µl of 1 × PBS and centrifuged at 70 - 100 × rcf for 1 minute. This step is performed four times in total.
7. 70 µl of elution buffer (IgG Elute Pierce) was added to each well and incubated for 1 min.
8. The plate was centrifuged at 70 - 100 × rcf for 1 minute to collect the eluate. This step is performed two times in total.

### Example 8: SPR Analysis of Antibody for Antigen Y

### AIM of experiment

A SPR analysis was performed to determine the binding affinity and the kinetics of interaction of the Hit 1 (ie, a lambda-type antibody from our common light chain mouse) to Target Y. The affinity and the kinetics of the purified antibody was compared to the benchmark and an isotype control (ISTC).

### Methods

Surface plasmon resonance (SPR) was used to determine the binding affinity (K_{D}) to the antigen Y, the kinetic constants on-rate (kₒₙ) and off-rate (k_{off}). The analysis was performed using a Biacore 8K (GE Healthcare) system.

An anti-human IgG Fc antibody was immobilised on CM4 chip (GE Healthcare catalogue number BR100534) according to manufacturer's instructions. Amine coupling kit (BioRad) was used to activate the surface of the chip. The surface was subsequently blocked with 1M ethanolamine. The immobilisation run was performed at 25°C using HBS-EP as immobilisation running buffer.

The purified monospecific antibodies (referred as ligand) from Example 7 were captured onto the anti-human IgG Fc CM4 surface at approximately 2ug/ml. The ligands were injected for 60 seconds at 10 ul/min in all the active channels of all 8 flow channels. The run was performed at 25°C using neutral pH HBS-P 1X + CaCl2 2.5 mM as running buffer.

Human antigen Y was reconstituted at 1mg/ml in the running buffer and used as analyte. The antigen Y was injected in multiple cycle kinetics (MCK) mode at 3 concentrations (1.5uM, 500nM and 166.7 nM) with 120 seconds association phase and 200 seconds dissociation phase, at flow rate 30ul/sec in both active and reference channels. Three injections of 10 mM Glycine pH 1.5 for 60 sec. at 10 µl/min were used for the regeneration phase.

An isotype control antibody hIgG4PE KYAB2229 (ISTC; this does not specifically bind antigen Y) was captured at 1ug/ml for 60 sec at 10 µl/min in the reference channel. An isotype control antibody hIgG4PE was also captured in the active channel as a negative control. Two antibodies against antigen Y, were used as benchmarks (Benchmark 1 and Benchmark 2). The data were reference and buffer subtracted and fitted into Langmuir 1:1 model. The first 30 seconds of dissociation were evaluated in the model.

**Table 2. Binding affinity and kinetic constants on-rate (kₒₙ) and off-rate (k_{off}) of anti-antigen Y antibodies**

| **Captured Antibody** | **kₒₙ (1/Ms)** | **k_{off} (1/s)** | **K_{D} (M)** |
|---|---|---|---|
| Benchmark 1 | 2.94 X 10⁴ | 4.27 X 10⁻² | 1.45 X 10 ⁻⁶ |
| Benchmark 2 | 4.13 X 10⁴ | 2.72 X 10⁻² | 6.60 X 10⁻⁷ |
| Antibody Hit 1 | 1.19 X 10⁵ | 1.25 X 10⁻¹ | 1.05 X 10⁻⁶ |
| hIgG4PE ISTC | | | NB |

The association and dissociation data of the interaction were fitted using biomolecular reaction model (1:1 Langmuir model). The values for association rate constant (kₒₙ), dissociation rate constant (k_{off}) and dissociation constant (K_{D}) were calculated from the binding data by BIAevaluation software.
Benchmark 1: antibody anti-antigen Y
Benchmark 2: antibody anti-antigen Y
NB: no binding observed
hIgG4PE ISTC: human isotype control IgG4

Hit 1 antibody was in IgG4PE format.

### Conclusion

Hit 1 showed binding to antigen Y in the affinity range provided in Table 2 and fast association (kₒₙ) and dissociation (k_{off}) rates for antigen Y. No binding to antigen Y was observed with ISTC.

Hit 1 antibody shows similar kinetic rates and binding affinity to antigen Y compared to the benchmark antibodies. This shows that our common light chain mice can generate antibodies with human variable domains that specifically bind to target antigen with desirable binding kinetics.

### Example 9: Analysis of B-Cell Populations from Common Light Chain Mice

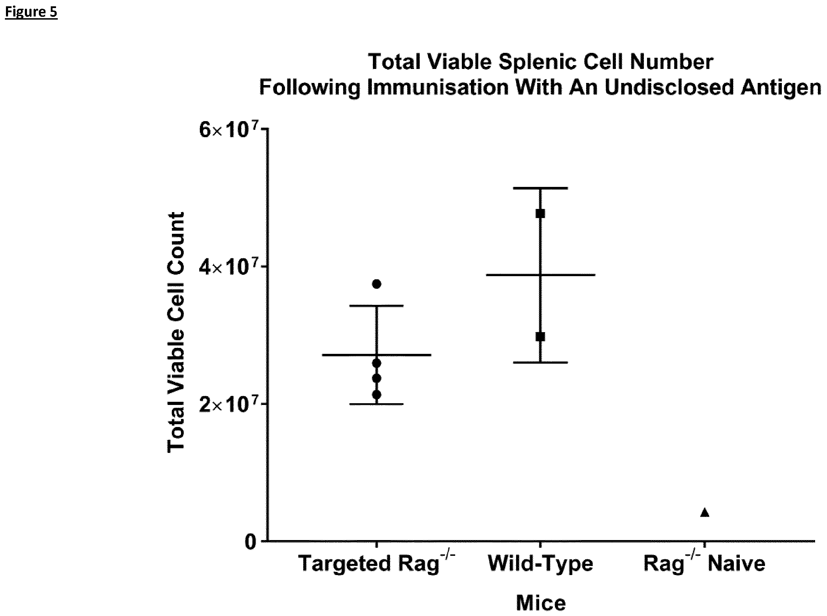

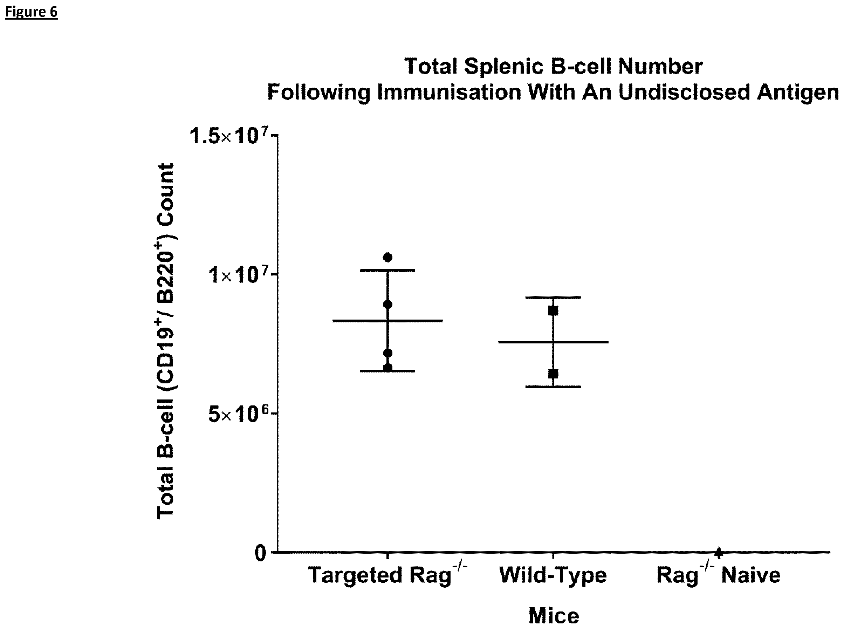

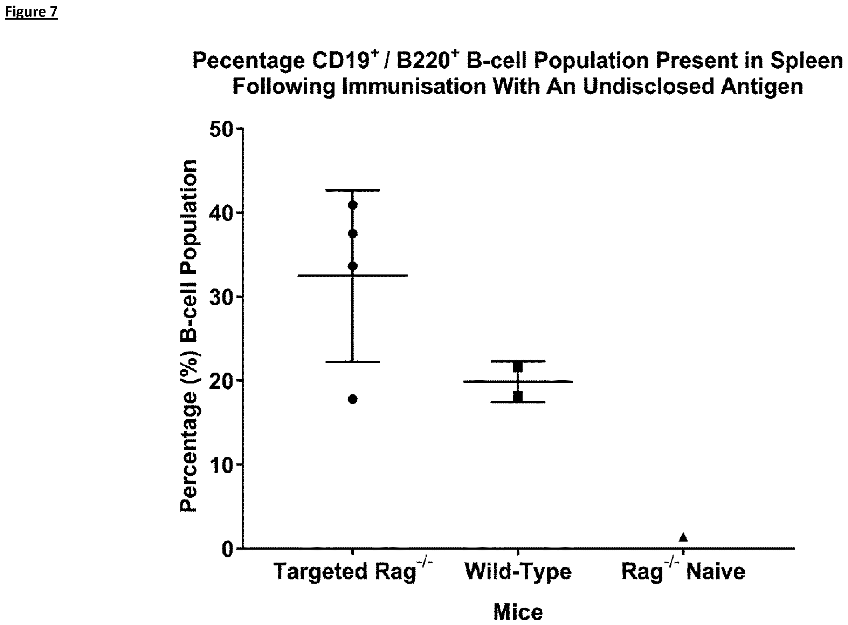

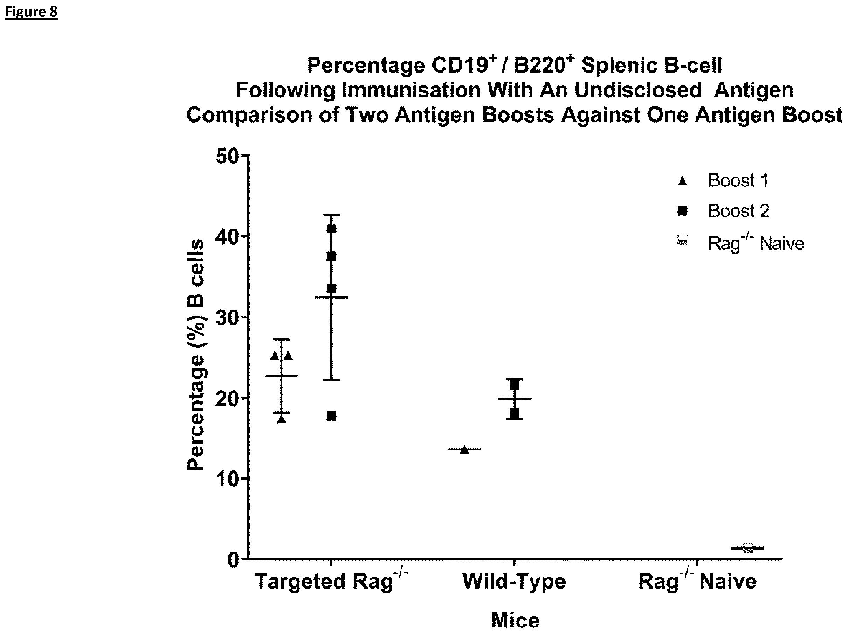

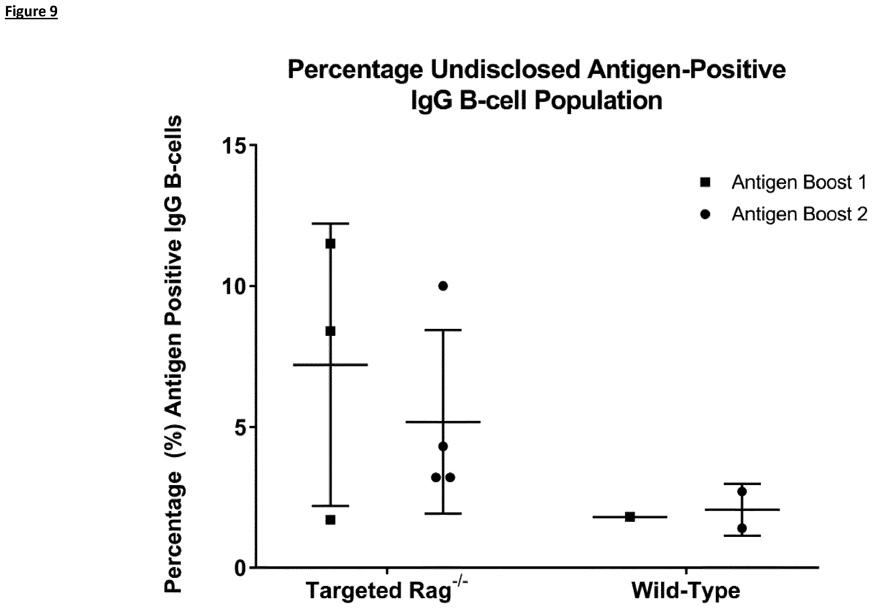

We analysed the B-cell compartments of mice of the invention. The results are shown in figures 5 to 9 as follows. "Targeted Rag^{-/-}" mice were mice produced as described in Example 4, derived from engineered ES cells. Thus, the genomes of the mice comprised the rearranged human lambda light chain knock-in and an unrearranged human heavy chain variable region comprised by a IgH locus.

### Figure 5

Chimera mice generated from Rag^{-/-} blastocysts micro-injected with targeted ES cells (targeted Rag^{-/-}) had similar total viable spleen cell number compared to wild-type mice (Wild-Type). Total viable spleen cell number following immunisation (antigen prime followed by two antigen boosts) with undisclosed antigen (Target Y). As a control, un-immunised Rag^{-/-} deficient mice displayed low total viable spleen cell number. Targeted Rag^{-/-}, chimera mouse derived from targeted ES-cells and Rag^{-/-} deficient blastocysts; Wild-Type, wild-type mouse; Rag^{-/-} Naive, un-immunised Rag^{-/-} deficient mice.

### Figure 6

Chimera mice generated from Rag^{-/-} blastocysts micro-injected with targeted ES cells (targeted Rag^{-/-}) had similar CD19-postive, B220-positve B-cell number compared to wild-type mice (Wild-Type). Total splenic B-cell number following immunisation (antigen prime followed by two antigen boosts) with undisclosed antigen (Target Y). As a control, un-immunised Rag^{-/-} deficient mice (Rag^{-/-} Naive) displayed minimal total B-cell (CD19⁺/ B220⁺) number. Targeted Rag^{-/-}, chimera mouse derived from targeted ES-cells and Rag^{-/-} deficient blastocysts; Wild-Type, wild-type mouse; Rag^{-/-} Naive, un-immunised Rag^{-/-} deficient mice.

### Figure 7

Percentage CD19-positive, B220-positive splenic B-cell population in chimera mice generated from Rag^{-/-} blastocysts micro-injected with targeted ES cells (targeted Rag^{-/-}) was similar to wild-type mice (Wild-Type). Percentage CD19-positive, B220-positive splenic B-cell population following immunisation (antigen prime followed by two antigen boosts) with undisclosed antigen (Target Y). As a control, un-immunised Rag^{-/-} deficient mice (Rag^{-/-} Naive) displayed minimal percentage CD19-positive, B220-positive splenic B-cell population. Targeted Rag^{-/-}, chimera mouse derived from targeted ES-cells and Rag^{-/-} deficient blastocysts; Wild-Type, wild-type mouse; Rag^{-/-} Naive, un-immunised Rag^{-/-} deficient mice.

### Figure 8

Percentage CD19-positive, B220-positive splenic B-cell population in chimera mice generated from Rag^{-/-} blastocysts micro-injected with targeted ES cells (targeted Rag^{-/-}) was similar to wild-type mice (Wild-Type). Percentage CD19-positive, B220-positive splenic B-cell population following immunisation (antigen prime followed by either one or two antigen boosts) with undisclosed antigen (Target Y). As a control, un-immunised Rag^{-/-} deficient mice (Rag^{-/-} Naive) displayed minimal percentage CD19-positive, B220-positive splenic B-cell population. Targeted Rag^{-/-}, chimera mouse derived from targeted engineered ES-cells and Rag^{-/-} deficient blastocysts; Wild-Type, wild-type mouse; Rag^{-/-} Naive, un-immunised Rag^{-/-} deficient mice.

### Figure 9

Percentage antigen positive IgG B-cell population in chimera mice generated from Rag^{-/-} blastocysts micro-injected with targeted ES cells (targeted Rag^{-/-}) was similar to wild-type mice (Wild-Type). Percentage antigen positive, CD19-positive or B220-positive, IgM-negative and IgD-negative splenic B-cell population following immunisation (antigen prime followed by either one or two antigen boosts) with undisclosed antigen (Target Y). Targeted Rag^{-/-}, chimera mouse derived from targeted engineered ES-cells and Rag^{-/-} deficient mice; Wild-Type, wild-type mouse.

### Conclusion

"Targeted Rag^{-/-}" chimera mice derived from engineered ES cells micro-injected into Rag^{-/-} deficient blastocysts produced IgM-negative, IgD-negative, antigen-positive splenic B-cells following an antigen prime and either one or two antigen boosts in a manner similar to wild-type mice. In comparison, non-chimeric Rag^{-/-} deficient mice did not produce splenic B-cells. These observations support our observation that mice generated from engineered ES cells micro-injected into Rag^{-/-} blastocysts have a functional immune response which generated antigen positive IgG B-cells in response to an immunisation regime.

Usefully, a respective copy of the common light chain can be paired with a copy of a heavy chain from an antibody (isolated from an immunised common light chain mouse of the invention) that binds to Target Y and a copy of a heavy chain from an antibody that binds Target Z to produce a bispecific 4-chain antibody that specifically binds Y and Z. This can be expressed from a cell (eg, a eukaryotic, mammalian, human, CHO, Cos or HEK293 cell) that comprises (i) an expressible nucleotide sequence encoding the light chain; (ii) an expressible nucleotide sequence encoding a heavy chain that specifically binds Target Y; and (iii) an expressible nucleotide sequence encoding a heavy chain that specifically binds Target Z. Alternatively, the cell comprises (i) and (ii) and a second cell comprises (iii), wherein the heavy and light chain expression products of the cells can be mixed to produce the bispecific antibody. Alternatively, the cell comprises (i) and (iii) and a second cell comprises (ii), wherein the heavy and light chain expression products of the cells can be mixed to produce the bispecific antibody. Alternatively, the cell comprises (ii) and (iii) and a second cell comprises (i), wherein the heavy and light chain expression products of the cells can be mixed to produce the bispecific antibody. Alternatively, (i), (ii) and (iii) are comprised by separate, respective cells and the antibody chain expression products of the cells are mixed to produce a bispecific antibody. In an example, the heavy chain constant regions comprise motifs or mutations (eg, charge pairs or knobs-in-holes motifs, see references above) that promote pairing of the anti-Target Y heavy chain with the anti-Target Z heavy chain.

### Methodology

### Introduction:

Following immunisation with antigen Y, splenic B-cells expressing class switched antibodies specific for said antigen were processed and single cell sorted. Targeted Rag^{-/-} chimera mice were analysed for evidence of a functional immune system and were compared to wild-type control mice. An additional comparison was also made to non-chimeric Rag^{-/-} mice, which demonstrate a minimal baseline immune response.

### Materials :

**Table 3: Table of reagents:**

| **Reagent** | **Supplier** | **Cat #** | **Lot #** |
|---|---|---|---|
| RPMI | Gibco | 11835-063 | 1881906 |
| Heat-inactivated Foetal Bovine Serum | Gibco | 10270-106 | 08G2073K |
| RNA Lysis buffer | Epicentre | QER090150 | 20151690 |
| TruStain fcX/Fc blocker | Biolegend | 101320 | B221805 |
| 7-AAD | eBioscience | 00-6993-50 | 4298663 |
| CD19-BUV395 | BD Horizon | 563557 | 7125716 |
| CD45R/B220-BUV395 | BD Horizon | 563793 | 7177756 |
| IgM-BV786 | BD Horizon | 564028 | 7048650 |
| IgD-BV605 | BD Horizon | 563003 | 7226860 |
| Ly-6G-APCCy7 | BD Pharmingen | 557661 | 7040615 |
| CD8a-APC-H7 | BD Pharmingen | 560182 | 7110593 |
| F4/80-APCCy7 | Biolegend | 123118 | B217243 |
| CD4-APC-H7 | BD Pharmingen | 560181 | 7012678 |
| CD11c-APC-Cy7 | BD Pharmingen | 561241 | 7096608 |
| ACK Lysis Buffer | Life Technologies | A10492-01 | N/A |
| Undisclosed antigen Y-647 (1 µg/ml) | In-house | | |

### Method:

### Processing of samples:

Spleens from immunised mice were removed and homogenised using 40 µm cell strainers using aseptic procedures. Cells were pelleted by centrifugation (350 rcf for 10 minutes at 4 °C) and the supernatant was carefully aspirated. Red blood cells were removed by lysis using 1 ml of ACK lysis buffer and incubated for 2 minutes at room-temperature. The lysis reaction was terminated by adding an excess of 3 % FBS / RPMI buffer. Cells were subsequently filtered using 40 µm cell strainers, pelleted as before and resuspended in 340 µl buffer. Any Fc receptors expressed on processed cells were blocked, removing non-specific staining of B-cells whilst maintaining antibody-mediated specific staining, using Fc blocker. At this stage a total cell count was taken.

### Staining:

Single-cell suspensions prepared from spleens were stained in the following manner. Mature, class-switched B-cell spleen populations were defined using BUV395-conjugated anti-CD45R (selecting), BUV395-conjugated anti-CD19 (selecting), BV786-conjugated anti-IgM (depleting) and BV605-conjugated anti-IgD (depleting). Cells other than B-cell such as neutrophils and monocytes, CD8-postive T-cells, macrophages, CD4 positive T-cells and dendritic cells were excluded using APCCy7-conjugated anti-Ly-6G/C, APC-H7-conjugated anti-CD8a, APCCy7-conjugated anti-F4/80, APC-H7-conjugated anti-CD4 and APC-Cy7-conjugated anti-CD11c, respectively. Antigen positive populations were identified using the same antigen used for immunisation labelled with Alexa-647. Non-viable cells were excluded using 7-AAD. An antigen-unlabelled control pool of cells was created to allow a FMO (Fluorescence Minus One) to be derived. Additional positive staining controls and 7AAD (live/dead) controls were also taken. All staining reactions were incubated at 4 °C for 20 min using 1 × 10⁶ cells in 100 µl of PBS with 3% FBS. Samples were washed twice in 400 µl PBS with 3% FBS and analyzed on BD FACSAria Fusion flow cytometer (BD Biosciences). All acquired data were analyzed using Flow-Jo^{™} software. Cells of interest were single cell sorted directly into 96 well plates containing RNA lysis buffer. Samples were then processed using B-Cell Technology (BCT).

## Claims

1. A cell comprising an antibody kappa light chain locus (first locus), wherein the locus comprises (in 5' to 3' direction)
(a) a light chain EiK intronic enhancer;
(b) a rearranged antibody variable region encoding a rearranged antibody V domain; and
(c) an antibody light chain constant region encoding a light chain C domain;
wherein the locus is operable to express an antibody chain comprising (in N- to C-terminal direction) said rearranged V domain and said C domain,
wherein the cell genome comprises a second antibody locus, wherein the second locus is an unrearranged antibody heavy chain locus comprising (in 5' to 3' direction)
(a) VH gene segments;
(b) one or more DH gene segments;
(c) one or more JH gene segments; and
(d) a heavy chain constant region encoding one or more CH domains;
wherein the heavy chain locus is operable to express a plurality of heavy chains; and
wherein said heavy chain locus comprises
(i) all human VH gene segments of the group consisting of IGHV1-3, IGHV1-8, IGHV1-18, IGHV1-46, IGHV3-7, IGHV3-9, IGHV3-11, IGHV3-15, IGHV3-20, IGHV3-21, IGHV3-23, IGHV3-30, IGHV3-33, IGHV3-48, IGHV3-53, IGHV4-4, IGHV4-31, IGHV4-34, IGHV4-39, IGHV4-59, IGHV4-61, IGHV5-51, IGHV6-1 and IGHV7-4-1; or
(ii) at least 5 human VH gene segments of the group consisting of IGHV1-3*01, IGHV1-8*01, IGHV1-18*01, IGHV1-46*03, IGHV3-7*01, IGHV3-9*01, IGHV3-11*01, IGHV3-15*01, IGHV3-20*d01, IGHV3-21*03, IGHV3-23*04, IGHV3-30*18, IGHV3-33*01, IGHV3-48*02, IGHV3-53*01, IGHV4-4*02, IGHV4-31*03, IGHV4-34*01, IGHV4-39*01, IGHV4-59*01, IGHV4-61*01, IGHV5-51*01, IGHV6-1*01 and IGHV7-4-1*01;

2. The cell of any preceding claim, wherein
(a) the rearranged V domain is a VK and the C domain is a CK;
(b) the rearranged V domain is a VK and the C domain is a CA;
(c) the rearranged V domain is a vλ and the C domain is a CA; or
(d) the rearranged V domain is a vλ and the C domain is a CK.

3. The cell of any preceding claim, wherein the locus comprises (in 5' to 3' direction)
(a) a promoter operable for promoting transcription of the variable region;
(b) a nucleotide sequence encoding a variable domain signal peptide;
(c) said intronic enhancer;
(d) said rearranged antibody variable region; and (e) said antibody light chain constant region,
Wherein the locus is operable to express RNA transcripts encoding (in N- to C-terminal direction) said signal peptide sequence fused to the amino acid sequence of said variable domain.

4. The cell of any preceding claim, wherein the cell is an ES, iPS, hybridoma or B-cell.

5. The cell of any preceding claim, wherein the rearranged V domain is a kappa or lambda V domain.

6. The cell of any preceding claim, wherein the variable region encodes for a V domain that has a binding specificity for a first predetermined antigen or a first epitope, wherein the locus is operable to express an antibody chain that comprises a V domain that retains said specificity.

7. The cell of any preceding claim, wherein the cell comprises a second antibody locus that is operable to express a second antibody chain, wherein the second chain comprises a second rearranged V domain that forms a binding site with the first rearranged V domain, wherein the binding site is capable of specifically binding to a predetermined antigen or epitope.

8. The cell of claim 7 when dependent from claim 6, wherein the predetermined antigens are different and said other and second V domains are different; or wherein the epitopes are different and said other and second V domains are different.

9. The cell of claim 8, wherein
(a) the first domain is a VL domain and the second and said other V domains are VH domains; or
(b) wherein the first domain is a VH domain and the second and said other V domains are VL domains.

10. The cell of any preceding claim, wherein the variable region is within 0.5 kb 3' of the enhancer.

11. The cell of any preceding claim, wherein the locus comprises a second enhancer that is 3' of the constant region.

12. The cell of any preceding claim, wherein the cell is a non-human mammal, mouse, rat or rodent cell.

13. The cell of any preceding claim, wherein said constant region is at an endogenous antibody locus of the cell.

14. The cell of claim 13 when dependent from claim 1, wherein said endogenous locus is an endogenous kappa chain locus.

15. The cell of any one of claims 1 to 12, wherein the locus is comprised by a transgene that is comprised by the genome of the cell at a position outside an endogenous antibody locus.

16. The cell of any preceding claim, wherein the cell is homozygous for said locus.

17. The cell of any preceding claim when dependent from claim 1, wherein the heavy chain locus is operable to express a plurality of heavy chains, comprising a plurality of antigen specificities or affinities, each said heavy chain being capable of pairing with an antibody chain encoded by the first locus to produce paired chains that comprise an antigen binding site.

18. The cell of any preceding claim, wherein each variable region or gene segment is human.

19. The cell of any preceding claim, wherein the cell is a mouse cell and each constant region is a mouse, rat or human constant region.

20. The cell of any preceding claim, wherein each said enhancer is an endogenous enhancer of the cell.

21. The cell of any preceding claim, wherein the cell is a mouse cell and each said enhancer is a mouse enhancer.

22. The cell of any preceding claim, wherein
(a) the cell is a mouse cell, the variable region is a human variable region, the intronic enhancer is a mouse intronic enhancer at an endogenous antibody locus of the cell and said constant region is a mouse, rat or human constant region; or
(b) the cell is a rat cell, the variable region is a human variable region, the intronic enhancer is a rat intronic enhancer at an endogenous antibody locus of the cell and said constant region is a mouse, rat or human constant region.

23. The cell of any preceding claim, wherein the rearranged variable region is a rearrangement of
(a) human IGLV3-21 and IGLJ3 and optionally operably connected to human germline IGLV3-21 promoter and/or signal peptide-encoding nucleotide sequence;
(b) human IGK1-39 and IGKJ1 or 5 and optionally operably connected to human germline IGKV1-39 promoter and/or signal peptide-encoding nucleotide sequence;
(c) human IGK3-20 and IGKJ1 or 5 and optionally operably connected to human germline IGKV3-20 promoter and/or signal peptide-encoding nucleotide sequence;
(d) a human VpreB and J\5 and optionally operably connected to human germline VpreB promoter and/or signal peptide-encoding nucleotide sequence.

24. A transgenic non-human vertebrate comprising a plurality of cells according to any preceding claim.

25. The vertebrate of claim 24, wherein the germline of said vertebrate comprises a first locus (and optionally the second locus) as defined in any one of claims 1 to 23.

26. The vertebrate of claim 24, wherein the vertebrate is a chimaera of said cells and a plurality of other cells that do not comprise the first locus, wherein the germline of the vertebrate does not comprise a said first locus.

27. The vertebrate of any one of claims 24 to 26, comprising a first plurality of cells and a second plurality of cells, wherein the cells of the first plurality comprise the same first identical antibody locus, and the cells of the second plurality comprise the same additional identical antibody locus, wherein each said antibody locus is according to the first locus of any one of claims 1 to 27, wherein the vertebrate is capable of expressing a first plurality of antibody chains from said first identical locus and a second plurality of antibody chains from said additional locus, and wherein the antibody locus of the first cells is different from the antibody locus of the second cells.

28. The vertebrate of claim 27, wherein the vertebrate comprises a third plurality of cells, wherein the cells of the third plurality comprise the same further antibody locus, wherein the further locus is according to any one of claims 1 to 23 and is different from said antibody loci of the first and second cells, wherein the vertebrate is capable of expressing a third plurality of antibody chains from the further locus.

29. The vertebrate of claim 27 or 28, wherein the vertebrate is a chimaera of said cells and a plurality of other cells that do not comprise a first locus according to any one of claims 1 to 24 or said additional or further loci.

30. The vertebrate of any one of claims 24-29, wherein the antibody light chain repertoire is at least 99% pure for a single rearranged VL domain species.

31. A population of spleen, bone marrow, B-cells, hybridomas, CHO cells, HEK cells, MEF cells, COS cells, HeLa cells or blood cells, wherein each cell is according to any one of claims 1-23; and optionally, wherein the cells express at least 10 different antibody species wherein
(a) at least 95% of the antibodies share the same light chain VL domain and the population comprises at least 10 different VH domain species; or
(b) the antibodies comprise VL domains derived from the recombination of a first VL gene segment and a first JL gene segment, wherein at least 95% of all said VL domains derived from said recombination comprise the same VL amino acid sequence and the population comprises at least 10 different VH domain species.

32. A method for identifying or obtaining an antibody, an antibody variable domain, a nucleotide sequence encoding an antibody or an antibody variable domain, or an expression vector or host cell that is capable of expressing the antibody or domain, wherein the antibody or domain is capable of specifically binding to a target antigen, the method comprising
(a) contacting the cell population of claim 31 with said antigen;
(b) binding antibodies expressed or comprised by said population to said antigen; and
(c) isolating or identifying one or more antibodies that bind to the antigen, or isolating or identifying a VH and/or a VL domain of a said one or more antibody; or identifying a nucleotide sequence encoding a said VH or VL; and
(d) optionally
(i) correlating a said identified antibody or domain with a nucleotide sequence encoding therefor, thereby identifying said sequence;
(ii) amplifying said sequence and inserting the sequence into an expression vector or a host cell genome for expression of the encoded antibody or domain; and
(iii) optionally expressing and isolating said antibody or domain,
wherein steps (i) and (ii) can be carried out in any order.

33. The method of claim 32, comprising using the method to amplify the VH and VL nucleotide sequences encoding the VH and VL domains of the identified antibody and further comprising inserting the VH and VL nucleotide sequences into the vector or cell for co-expression of the VH and VL to produce paired VH/VL binding sites that are capable of binding to the antigen, and expressing (and optionally isolating) the binding sites.

34. The method of claim 33, wherein the method comprises expressing the VH and VL in the presence of a further species of VH domain, wherein the VL and the further VH form further paired VH/VL sites that are capable of binding to a further antigen, wherein the further antigen and the antigen recited in claim 33 are different, the method comprising co-expressing (and optionally isolating) the binding sites wherein the binding sites are comprised by bi-specific antibodies encoded by the vector or cell, wherein the bi-specific antibodies comprise a respective binding site for each said antigen.

35. The method of claim 34, wherein the bi-specific antibodies are 4-chain antibodies comprising a first heavy chain-light chain pair and a second heavy chain-light chain pair, wherein
(a) the light chain in the first and second pairs comprises said VL domain,
(b) the heavy chain of the first pair comprises the VH domain recited in claim 34, wherein the VH domain forms a VH/VL binding site with said VL domain and wherein the binding site is capable of specifically binding to said antigen recited in claim 33;
(c) the heavy chain of the second pair comprises said further VH domain that forms a VH/VL binding site with said VL domain and wherein the binding site is capable of specifically binding to said further antigen recited in claim 34.

36. The method of claim 35, wherein each 4-chain antibody comprises mutated heavy chain constant regions to promote pairing of the heavy chains of the first and second heavy-light pairs; optionally wherein the mutations are knob-in-hole mutations or charge pair mutations.

37. The method of any one of claims 32 to 36, wherein the method comprises obtaining VL and/or VH-encoding nucleotide sequences from the cells of claim 31 or 35, and in step (d) (i) using the nucleotide sequences to correlate one or more thereof with one or more VH and/or VL domains of said antibodies that bind the antigen.

38. A method for obtaining a nucleotide sequence encoding an antibody or an antibody variable domain, wherein the antibody or domain is capable of specifically binding to a target antigen, the method comprising
(a) obtaining VL and/or VH-encoding nucleotide sequences from the cells of claim 31 or 35; (b) amplifying said sequence; and
(c) optionally inserting the sequence into an expression vector or a host cell genome for expression of the encoded antibody or domain.

39. A method for obtaining an antibody-producing cell line for the production of antibodies that specifically bind to an antigen, the method comprising obtaining VL and/or VH-encoding nucleotide sequences from the cells of claim 31 or 35; and inserting the sequences into the genome of a host cell, wherein
(a) each VH sequence is operably inserted 5' of an antibody heavy chain constant region for expression of heavy chains by the cell comprising VH and C domains;
(b) each VL sequence is operably inserted 5' of an antibody light chain constant region for expression of light chains by the cell comprising VL and C domains;
(c) wherein the expressed heavy chains are capable of pairing with the light chains to
produce heavy-light chain pairs, each pair comprising a VH/VL binding site that is capable of binding to an antigen; and
(d) wherein VH and VL-encoding nucleotide sequences are the sequences of VH and VL-encoding nucleotide sequences of one or more cells of claim 31.

40. The method of claim 39, comprising inserting first and second VH-encoding nucleotide sequences into the cell genome, wherein
(a) the first VH (when expressed as part of first heavy chains) is capable of pairing with the VL (when expressed as part of light chains) to form a first VH/VL binding site that is capable of specifically binding to a first antigen;
(b) the second VH (when expressed as part of second heavy chains) is capable of pairing with the VL (when expressed as part of light chains) to form a second VH/VL binding site that is capable of specifically binding to a second antigen; and
(c) the first and second antigens are different;
wherein the first and second heavy chains and the light chains are capable of pairing to produce bi- specific 4-chain antibodies comprising a first heavy chain-light chain pair and a second heavy chain- light chain pair, wherein
(d) the heavy chain of the first heavy chain-light chain pair comprises the first VH; and
(e) the heavy chain of the second heavy chain-light chain pair comprises the second VH domain.

41. A method for obtaining a bispecific antibody, the method comprising:
1. Obtaining a first antibody comprising first human VH and VL domains, wherein the antibody specifically binds to a first predetermined antigen; or obtaining nucleotide sequences encoding said VL or the light chain of the antibody;
2. Obtaining a transgenic mouse ES cell comprising an IgH locus (in heterozygous or homozygous form), wherein the IgH locus comprises a humanised variable region for expressing a plurality of different human VH domains;
3. Inserting a nucleotide sequence encoding the light chain of the antibody or the VL thereof into the genome of the ES cell, wherein the insertion is between the EiK and CK of a kappa locus allele of the ES cell genome to produce a cell according to any one of claims 1-23;
4. Optionally modifying the other kappa locus allele so that it is not capable of expressing kappa light chains, or modifying the allele whereby a nucleotide sequence encoding the light chain of the first or a second antibody or the VL thereof is inserted between the EiK and CK of said other kappa locus allele;
5. Developing the ES cell into a mouse, wherein the mouse or pup(s) so
developed comprises at least one kappa locus that is capable of expressing the light chain or VL, wherein the light chain or VL pair with heavy chains or VH expressed by the mouse to form VH/VL antigen binding sites;
6. Optionally obtaining progeny mice, wherein the progeny each comprise at least one kappa locus that is capable of expressing the light chain or VL, wherein the light chain or VL pair with heavy chains or VH expressed by the progeny mouse to form VH/VL antigen binding sites;
7. Immunising the mouse of step 5 or a said progeny mouse with a second antigen, wherein the first and second antigens are different;
8.Obtaining from an immunised mouse of step 7 a nucleotide sequence encoding a VH (second VH) that pairs with the VL (or a VH of a heavy chain that pairs with the VL of the light chain), wherein the second VH and VL form an antigen binding site that specifically binds to the second antigen;
9. Inserting a nucleotide sequence encoding the first VH, a nucleotide sequence encoding the second VH, and a nucleotide sequence encoding the VL into one or more expression vectors or into the genome of one or more host cells, each V nucleotide sequence being in operable connection 5' of an antibody first CH, second CH or CL region respectively for expression of first antibody heavy chains, second antibody heavy chains or antibody light chains respectively; optionally wherein the first and second CH regions encode first and second CH domains that are mutated human CH domains that pair together and/or wherein the first and second chains have different pl;
10. Obtaining host cells of step 9 and expressing the heavy and light chains in the host cells, whereby bi-specific antibodies are produced each comprising a first heavy chain/light chain pair comprising a VH/VL binding site that is capable of specifically binding to the first antigen, and a second heavy chain/light chain pair comprising a VH/VL binding site that is capable of specifically binding to the second antigen; and
11. Isolating said bi-specific antibodies.

42. A method for producing a cell of any one of claims 1 to 23 or vertebrate of any one of claims 24 to 30, the method comprising
(a) obtaining a nucleic acid comprising a rearranged antibody variable region encoding a rearranged antibody V domain; and
(b) inserting the variable region or a copy thereof into the genome of a cell, whereby the variable region is comprised by an antibody locus in said genome,
wherein the locus comprises (in 5' to 3' direction) (c) an antibody light chain EiK intronic enhancer;
(d) said rearranged antibody variable region; and
(e) an antibody constant region encoding an antibody light chain C domain;
wherein the locus is operable to express an antibody chain comprising (in N- to C-terminal direction) said rearranged V domain and said C domain; and
(f) optionally, wherein the cell is a non-human vertebrate ES cell or an iPS cell, the method further comprising generating the non-human vertebrate of any one of claims 24 to 30 from the cell.

43. The method of claim 42, wherein the variable domain is a VL and the C domain is CL.

44. The method of claim 42 or 43, wherein said inserting is carried out using homologous recombination or site-specific recombination with chromosomal DNA of the cell.

45. The method of any one of claims 42 to 44, comprising
obtaining a transgene comprising (in 5' to 3' direction)
(a) said EiK intronic enhancer;
(b) said rearranged antibody variable region; and
(c) said antibody light chain constant region; and
Inserting said transgene into the genome of the cell, whereby said antibody locus is comprised by the cell.

46. The method of any one of claims 42 to 44, comprising inserting said variable region between
(a) the intronic enhancer and the CL of a light chain locus of the cell genome; or
(b) the intronic enhancer (Eµ) and the Cµ of a heavy chain locus of the cell genome.

47. The method of any one of claims 42 to 46, wherein the variable region encodes a V domain of an antibody that specifically binds to a predetermined antigen.

48. The method of claim 47, wherein the step of obtaining the nucleic acid comprising the rearranged antibody variable region as recited in claim 42 comprises
(a) obtaining a further cell that expresses said antibody recited in claim 47;
(b) obtaining or copying
i. a DNA sequence of the further cell that comprises a rearranged variable region sequence encoding the V domain; or
ii. a NA sequence of the further cell that comprises a rearranged variable region sequence encoding the V domain, and creating a DNA copy of said RNA sequence;
thereby obtaining said nucleic acid.

49. The method of any one of claim 42 to 48, comprising culturing the resultant cell to produce a plurality of cells, wherein each cell is according to any one of claims 1 to 23.

50. A non-human vertebrate blastocyst or pre-morula embryo implanted with an ES or iPS cell according to any one of claims 1 to 23 or obtained according to the method of any one of claims 42 to 49.

51. A nucleic acid comprising a transgene for use in the method of claim 45, the transgene comprising (in 5' to 3' direction)
(a) said EiK intronic enhancer;
(b) said rearranged antibody variable region; and
(c) said antibody light chain constant region.

## Patentansprüche

1. Zelle, umfassend einen Antikörper-Kappa-Leichtketten-Locus (ersten Locus), wobei der Locus (in 5'-3'-Richtung) Folgendes umfasst:
(a) einen Leichtketten-EiK-Intron-Enhancer;
(b) eine umgeordnete variable Antikörper-Region, die eine umgeordnete Antikörper-V-Domäne codiert; und
(c) eine konstante Antikörper-Leichtketten-Region, die eine Leichtketten-C-Domäne codiert;
wobei der Locus dazu betriebsfähig ist, eine Antikörper-Kette zu exprimieren, die (in N-C-Terminus-Richtung) die umgeordnete V-Domäne und die C-Domäne umfasst,
wobei das Zellgenom einen zweiten Antikörper-Locus umfasst, wobei der zweite Locus ein nicht umgeordneter Antikörper-Schwerketten-Locus ist, der (in 5'-3'-Richtung) Folgendes umfasst:
(a) VH-Gen-Segmente;
(b) ein oder mehrere DH-Gen-Segmente;
(c) ein oder mehrere JH-Gen-Segmente; und
(d) eine konstante Schwerketten-Region, die eine oder mehrere CH-Domänen codiert;
wobei der Schwerketten-Locus dazu betriebsfähig ist, eine Vielzahl von Schwerketten zu exprimieren; und
wobei der Schwerketten-Locus Folgendes umfasst:
(i) alle menschlichen VH-Gen-Segmente der Gruppe bestehend aus IGHV1-3, IGHV1-8, IGHV1-18, IGHV1-46, IGHV3-7, IGHV3-9, IGHV3-11, IGHV3-15, IGHV3-20, IGHV3-21, IGHV3-23, IGHV3-30, IGHV3-33, IGHV3-48, IGHV3-53, IGHV4-4, IGHV4-31, IGHV4-34, IGHV4-39, IGHV4-59, IGHV4-61, IGHV5-51, IGHV6-1 und IGHV7-4-1; oder
(ii) mindestens 5 menschliche VH-Gen-Segmente der Gruppe bestehend aus IGHV1-3*01, IGHV1-8*01, IGHV1-18*01, IGHV1-46*03, IGHV3-7*01, IGHV3-9*01, IGHV3-11*01, IGHV3-15*01, IGHV3-20*d01, IGHV3-21*03, IGHV3-23*04, IGHV3-30*18, IGHV3-33*01, IGHV3-48*02, IGHV3-53*01, IGHV4-4*02, IGHV4-31*03, IGHV4-34*01, IGHV4-39*01, IGHV4-59*01, IGHV4-61*01, IGHV5-51*01, IGHV6-1*01 und IGHV7-4-1*01.

2. Zelle nach einem der vorhergehenden Ansprüche, wobei
(a) die umgeordnete V-Domäne ein VK ist und die C-Domäne ein CK ist;
(b) die umgeordnete V-Domäne ein VK ist und die C-Domäne ein Cλ ist;
(c) die umgeordnete V-Domäne ein vλ ist und die C-Domäne ein Cλ ist; oder
(d) die umgeordnete V-Domäne ein vλ ist und die C-Domäne ein CK ist.

3. Zelle nach einem der vorhergehenden Ansprüche, wobei der Locus (in 5'-3'-Richtung) Folgendes umfasst:
(a) einen Promotor, der zum Fördern der Transkription der variablen Region betriebsfähig ist;
(b) eine Nukleotid-Sequenz, die ein Signalpeptid einer variablen Domäne codiert;
(c) den Intron-Enhancer;
(d) die umgeordnete variable Antikörper-Region; und
(e) die konstante Antikörper-Leichtketten-Region,
wobei der Locus dazu betriebsfähig ist, RNA-Transkripte zu exprimieren, die (in N-C-Terminus-Richtung) die Signalpeptid-Sequenz codiert, welche an die Aminosäure-Sequenz der variablen Domäne fusioniert ist.

4. Zelle nach einem der vorhergehenden Ansprüche, wobei die Zelle eine ES-, iPS-, Hybridom- oder B-Zelle ist.

5. Zelle nach einem der vorhergehenden Ansprüche, wobei die umgeordnete V-Domäne eine Kappa- oder Lambda-V-Domäne ist.

6. Zelle nach einem der vorhergehenden Ansprüche, wobei die variable Region eine V-Domäne codiert, die eine Bindungsspezifität für ein erstes vordefiniertes Antigen oder ein erstes Epitop aufweist, wobei der Locus dazu betriebsfähig ist, eine Antikörper-Kette zu exprimieren, die eine V-Domäne umfasst, welche die Spezifität beibehält.

7. Zelle nach einem der vorhergehenden Ansprüche, wobei die Zelle einen zweiten Antikörper-Locus umfasst, der dazu betriebsfähig ist, eine zweite Antikörper-Kette zu exprimieren, wobei die zweite Kette eine zweite umgeordnete V-Domäne umfasst, die eine Bindungsstelle mit der ersten umgeordneten V-Domäne bildet, wobei die Bindungsstelle in der Lage ist, sich spezifisch an ein vorbestimmtes Antigen oder Epitop zu binden.

8. Zelle nach Anspruch 7 bei Abhängigkeit von Anspruch 6, wobei die vorbestimmten Antigene unterschiedlich sind und die andere und die zweite V-Domäne unterschiedlich sind; oder wobei die Epitope unterschiedlich sind und die andere und die zweite V-Domäne unterschiedlich sind.

9. Zelle nach Anspruch 8, wobei
(a) die erste Domäne eine VL-Domäne ist und die zweite und die andere V-Domäne VH-Domänen sind; oder
(b) die erste Domäne eine VH-Domäne ist und die zweite und die andere V-Domäne VL-Domänen sind.

10. Zelle nach einem der vorhergehenden Ansprüche, wobei sich die variable Region innerhalb von 0,5 kb 3' des Enhancers befindet.

11. Zelle nach einem der vorhergehenden Ansprüche, wobei der Locus einen zweiten Enhancer umfasst, der 3' der konstanten Region ist.

12. Zelle nach einem der vorhergehenden Ansprüche, wobei die Zelle eine nichtmenschliche Säugetier-, Maus-, Ratten- oder Nagetierzelle ist.

13. Zelle nach einem der vorhergehenden Ansprüche, wobei sich die konstante Region an einem endogenen Antikörper-Locus der Zelle befindet.

14. Zelle nach Anspruch 13 bei Abhängigkeit von Anspruch 1, wobei der endogene Locus ein endogener Kappa-Ketten-Locus ist.

15. Zelle nach einem der Ansprüche 1 bis 12, wobei der Locus in einem Transgen enthalten ist, das in dem Genom der Zelle an einer Position außerhalb eines endogenen Antikörper-Locus enthalten ist.

16. Zelle nach einem der vorhergehenden Ansprüche, wobei die Zelle für den Locus homozygot ist.

17. Zelle nach einem der vorhergehenden Ansprüche bei Abhängigkeit von Anspruch 1, wobei der Schwerketten-Locus dazu betriebsfähig ist, eine Vielzahl von Schwerketten zu exprimieren, umfassend eine Vielzahl von Antigen-Spezifitäten oder -Affinitäten, wobei jede Schwerkette in der Lage ist, sich mit einer Antikörper-Kette zu koppeln, die durch den ersten Locus codiert wird, um gekoppelte Ketten zu erzeugen, die eine Antigen-Bindungsstelle umfassen.

18. Zelle nach einem der vorhergehenden Ansprüche, wobei jede variable Region oder jedes Gensegment menschlich ist.

19. Zelle nach einem der vorhergehenden Ansprüche, wobei die Zelle eine Mauszelle ist und jede konstante Region eine konstante Maus-, Ratten- oder menschliche Region ist.

20. Zelle nach einem der vorhergehenden Ansprüche, wobei jeder Enhancer ein endogener Enhancer der Zelle ist.

21. Zelle nach einem der vorhergehenden Ansprüche, wobei die Zelle eine Mauszelle ist und jeder Enhancer ein Maus-Enhancer ist.

22. Zelle nach einem der vorhergehenden Ansprüche, wobei
(a) die Zelle eine Mauszelle ist, die variable Region eine menschliche variable Region ist, der Intron-Enhancer ein Maus-Intron-Enhancer an einem endogenen Antikörper-Locus der Zelle ist und die konstante Region eine konstante Maus-, Ratten- oder menschliche Region ist; oder
(b) die Zelle eine Rattenzelle ist, die variable Region eine menschliche variable Region ist, der Intron-Enhancer ein Ratten-Intron-Enhancer an einem endogenen Antikörper-Locus der Zelle ist und die konstante Region eine konstante Maus-, Ratten- oder menschliche Region ist.

23. Zelle nach einem der vorhergehenden Ansprüche, wobei die umgeordnete variable Region eine Umordnung
(a) eines menschlichen IGLV3-21 und IGLJ3 ist und optional mit einem IGLV3-21-Promotor der menschlichen Keimbahn und/oder einer ein Signalpeptid codierenden Nukleotid-Sequenz wirkverbunden ist;
(b) eines menschlichen IGK1-39 und IGKJ1 oder 5 ist und optional mit einem IGKV1-39-Promotor der menschlichen Keimbahn und/oder einer ein Signalpeptid codierenden Nukleotid-Sequenz wirkverbunden ist;
(c) eines menschlichen IGK3-20 und IGKJ1 oder 5 ist und optional mit einem IGKV3-20-Promotor der menschlichen Keimbahn und/oder einer ein Signalpeptid codierenden Nukleotid-Sequenz wirkverbunden ist;
(d) eines menschlichen VpreB und J\5 ist und optional mit einem VpreB-Promotor der menschlichen Keimbahn und/oder einer ein Signalpeptid codierenden Nukleotid-Sequenz wirkverbunden ist.

24. Transgener nichtmenschlicher Vertebrat, umfassend eine Vielzahl von Zellen nach einem der vorhergehenden Ansprüche.

25. Vertebrat nach Anspruch 24, wobei die Keimbahn des Vertebraten einen ersten Locus (und optional einen zweiten Locus), wie in einem der Ansprüche 1 bis 23 definiert, umfasst.

26. Vertebrat nach Anspruch 24, wobei der Vertebrat eine Chimäre der Zellen und einer Vielzahl von anderen Zellen ist, die nicht den ersten Locus umfassen, wobei die Keimbahn des Vertebraten den ersten Locus nicht umfasst.

27. Vertebrat nach einem der Ansprüche 24 bis 26, umfassend eine erste Vielzahl von Zellen und eine zweite Vielzahl von Zellen, wobei die Zellen der ersten Vielzahl von Zellen den gleichen ersten identischen Antikörper-Locus umfassen und die Zellen der zweiten Vielzahl von Zellen den gleichen zusätzlichen identischen Antikörper-Locus umfassen, wobei jeder Antikörper-Locus dem ersten Locus nach einem der Ansprüche 1 bis 27 entspricht,
wobei der Vertebrat in der Lage ist, eine erste Vielzahl von Antikörper-Ketten von dem ersten identischen Locus und eine zweite Vielzahl von Antikörper-Ketten von dem zusätzlichen Locus zu exprimieren, und wobei sich der Antikörper-Locus der ersten Zellen von dem Antikörper-Locus der zweiten Zellen unterscheidet.

28. Vertebrat nach Anspruch 27, wobei der Vertebrat eine dritte Vielzahl von Zellen umfasst, wobei die Zellen der dritten Vielzahl von Zellen den gleichen weiteren Antikörper-Locus umfassen, wobei der weitere Locus ein beliebiger nach einem der Ansprüche 1 bis 23 ist und sich von den Antikörper-Loci der ersten und zweiten Zellen unterscheidet, wobei der Vertebrat in der Lage ist, eine dritte Vielzahl von Antikörper-Ketten von dem weiteren Locus zu exprimieren.

29. Vertebrat nach Anspruch 27 oder 28, wobei der Vertebrat eine Chimäre der Zellen und einer Vielzahl von anderen Zellen ist, die keinen ersten Locus nach einem der Ansprüche 1 bis 24 oder keine zusätzlichen oder weiteren Loci umfassen.

30. Vertebrat nach einem der Ansprüche 24-29, wobei das Antikörper-Leichtketten-Repertoire für eine einzelne umgeordnete VL-Domänen-Spezies zu mindestens 99 % rein ist.

31. Population von Milz-, Knochenmark-, B-Zellen, Hybridomen, CHO-Zellen, HEK-Zellen, MEF-Zellen, COS-Zellen, HeLa-Zellen oder Blutzellen, wobei jede Zelle eine beliebige nach einem der Ansprüche 1-23 ist; und wobei die Zellen optional mindestens 10 unterschiedliche Antikörper-Spezies exprimieren, wobei
(a) mindestens 95 % der Antikörper die gleiche Leichtketten-VL-Domäne gemein haben und die Population mindestens 10 unterschiedliche VH-Domänen-Spezies umfasst; oder
(b) die Antikörper VL-Domänen umfassen, die von Rekombinationen eines ersten VL-Gen-Segments und eines ersten JL-Gen-Segments abgeleitet sind, wobei mindestens 95 % aller VL-Domänen, die von dieser Rekombination abgeleitet sind, die gleiche VL-Aminosäure-Sequenz umfassen und die Population mindestens 10 unterschiedliche VH-Domänen-Spezies umfasst.

32. Verfahren zum Identifizieren oder Erlangen eines Antikörpers, einer variablen Antikörper-Domäne, einer Nukleotid-Sequenz, die einen Antikörper oder eine variable Antikörper-Domäne codiert, oder eines Expressionsvektors oder einer Wirtszelle, der/die in der Lage ist, den Antikörper oder die Domäne zu exprimieren, wobei der Antikörper oder die Domäne in der Lage ist, sich spezifisch an ein Ziel-Antigen zu binden, wobei das Verfahren Folgendes umfasst:
(a) Inkontaktbringen der Zellpopulation nach Anspruch 31 mit dem Antigen;
(b) Binden von Antikörpern, die durch die Population exprimiert werden oder darin enthalten sind, an das Antigen; und
(c) Isolieren oder Identifizieren eines oder mehrerer Antikörper, die sich an das Antigen binden, oder Isolieren oder Identifizieren einer VH- und/oder einer VL-Domäne des einen oder der mehreren Antikörper; oder Identifizieren einer Nukleotid-Sequenz, die das VH oder VL codiert; und
(d) optional
(i) Korrelieren des identifizierten Antikörpers oder der identifizierten Domäne mit einer dafür codierenden Nukleotid-Sequenz, wodurch die Sequenz identifiziert wird;
(ii) Amplifizieren der Sequenz und Einfügen der Sequenz in einen Expressionsvektor oder ein Wirtszellengenom zur Expression des codierten Antikörpers oder der codierten Domäne; und
(iii) optional Exprimieren und Isolieren des Antikörpers oder der Domäne,
wobei Schritt (i) und (ii) ein eine beliebigen Reihenfolge ausgeführt werden können.

33. Verfahren nach Anspruch 32, umfassend Verwenden des Verfahrens, um die VH- und VL-Nukleotid-Sequenzen, die die VH- und VL-Domänen des identifizierten Antikörpers codieren, zu amplifizieren, und ferner umfassend Einfügen der VH- und VL-Nukleotid-Sequenzen in den Vektor oder die Zelle zur Co-Expression des VH und VL, um gekoppelte VH/VL-Bindungsstellen zu erzeugen, die in der Lage sind, sich an das Antigen zu binden, und Exprimieren (und optional Isolieren) der Bindungsstellen.

34. Verfahren nach Anspruch 33, wobei das Verfahren Exprimieren des VH und VL in Gegenwart einer weiteren VH-Domänen-Spezies, wobei das VL und das weitere VH weitere gekoppelte VH/VL-Stellen bilden, die in der Lage sind, ein weiteres Antigen zu binden, wobei sich das weitere Antigen und das in Anspruch 33 aufgeführte Antigen unterscheiden, wobei das Verfahren CoExprimieren (und optional Isolieren) der Bindungsstellen umfasst, wobei die Bindungsstellen in bispezifischen Antikörpern enthalten sind, die durch den Vektor oder die Zelle codiert werden, wobei die bispezifischen Antikörper eine jeweilige Bindungsstelle für jedes Antigen umfassen.

35. Verfahren nach Anspruch 34, wobei die bispezifischen Antikörper 4-Ketten-Antikörper sind, die ein erstes Schwerketten-Leichtketten-Paar und ein zweites Schwerketten-Leichtkettenpaar umfassen, wobei
(a) die Leichtkette in dem ersten und dem zweiten Paar die VL-Domäne umfasst,
(b) die Schwerkette des ersten Paares die in Anspruch 34 aufgeführte VH-Domäne umfasst, wobei die VH-Domäne eine VH/VL-Bindungsstelle mit der VL-Domäne bildet und wobei die Bindungsstelle in der Lage ist, sich spezifisch an das in Anspruch 33 aufgeführte Antigen zu binden;
(c) die Schwerkette des zweiten Paares die weitere VH-Domäne umfasst, die eine VH/VL-Bindungsstelle mit der VL-Domäne bildet, und wobei die Bindungsstelle in der Lage ist, sich spezifisch an das in Anspruch 34 aufgeführte weitere Antigen zu binden.

36. Verfahren nach Anspruch 35, wobei jeder 4-Ketten-Antikörper konstante Regionen mutierter Schwerketten umfasst, um ein Koppeln der Schwerketten des ersten und des zweiten Schwer-Leicht-Paares zu fördern; wobei die Mutationen optional Knauf-in-Loch-Mutationen oder Ladungspaar-Mutationen sind.

37. Verfahren nach einem der Ansprüche 32 bis 36, wobei das Verfahren Erlangen von VL und/oder VH codierenden Nukleotid-Sequenzen von den Zellen nach Anspruch 31 oder 35 und in Schritt (d) (i) Verwenden der Nukleotid-Sequenzen zum Korrelieren einer oder mehrerer davon mit einer oder mehreren VH- und/oder VL-Domänen der Antikörper, die das Antigen binden, umfasst.

38. Verfahren zum Erlangen einer Nukleotid-Sequenz, die einen Antikörper oder eine variable Antikörper-Domäne codiert, wobei der Antikörper oder die Domäne in der Lage ist, sich spezifisch an ein Ziel-Antigen zu binden, wobei das Verfahren Folgendes umfasst:
(a) Erlangen von VL- und/oder VH codierenden Nukleotid-Sequenzen von den Zellen nach Anspruch 31 oder 35;
(b) Amplifizieren der Sequenz; und
(c) optional Einfügen der Sequenz in einen Expressionsvektor oder ein Wirtszellengenom zur Expression des codierten Antikörpers oder der codierten Domäne.

39. Verfahren zum Erlangen einer Antikörper erzeugenden Zelllinie zur Erzeugung von Antikörpern, die sich spezifisch an ein Antigen binden, wobei das Verfahren Erlangen von VL- und/oder VH codierenden Nukleotid-Sequenzen von den Zellen nach Anspruch 31 oder 35 umfasst; und Einfügen der Sequenzen in das Genom einer Wirtszelle, wobei
(a) jede VH-Sequenz betriebsfähig 5' einer konstanten Region einer Antikörper-Schwerkette zur Expression von Schwerketten durch die Zelle, die VH- und C-Domänen umfasst, eingefügt wird;
(b) jede VL-Sequenz betriebsfähig 5' einer konstanten Region einer Antikörper-Leichtkette zur Expression von Leichtketten durch die Zelle, die VL- und C-Domänen umfasst, eingefügt wird;
(c) wobei die exprimierten Schwerketten in der Lage sind, sich mit den Leichtketten zu koppeln, um Schwer-Leichtketten-Paare zu erzeugen, wobei jedes Paar eine VH/VL-Bindungsstelle umfasst, die in der Lage ist, sich an ein Antigen zu binden; und
(d) VH- und VL codierende Nukleotid-Sequenzen die Sequenzen von VH und VL codierenden Nukleotidsequenzen einer oder mehrerer Zellen nach Anspruch 31 sind.

40. Verfahren nach Anspruch 39, umfassend Einfügen von ersten und zweiten VH codierenden Nukleotid-Sequenzen in das Zellgenom, wobei
(a) das erste VH (bei Expression als Teil von ersten Schwerketten) in der Lage ist, sich mit dem VL (bei Expression als Teil von Leichtketten) zu koppeln, um eine erste VH/VL-Bindungsstelle zu bilden, die in der Lage ist, sich spezifisch an ein erstes Antigen zu binden;
(b) das zweite VH (bei Expression als Teil von zweiten Schwerketten) in der Lage ist, sich mit dem VL (bei Expression als Teil von Leichtketten) zu koppeln, um eine zweite VH/VL-Bindungsstelle zu bilden, die in der Lage ist, sich spezifisch an ein zweites Antigen zu binden; und
(c) sich das erste und das zweite Antigen unterscheiden;
wobei die ersten und zweiten Schwerketten und die Leichtketten in der Lage sind, sich zu koppeln, um bispezifische 4-Ketten-Antikörper zu erzeugen, die ein erstes Schwerketten-Leichtketten-Paar und zweites Schwerketten-Leichtketten-Paar umfassen, wobei
(d) die Schwerkette des ersten Schwerketten-Leichtketten-Paares das erste VH umfasst; und
(e) die Schwerkette des zweiten Schwerketten-Leichtketten-Paares die zweite VH-Domäne umfasst.

41. Verfahren zum Erlangen eines bispezifischen Antikörpers, wobei das Verfahren Folgendes umfasst:
1. Erlangen eines ersten Antikörpers, der erste menschliche VH- und VL-Domänen umfasst, wobei sich der Antikörper spezifisch an ein erstes vorbestimmtes Antigen bindet; oder Erlangen von Nukleotidsequenzen, die das VL oder die Leichtkette des Antikörpers codieren;
2. Erlangen einer transgenen Maus-ES-Zelle, die einen IgH-Locus (in heterozygoter oder homozygoter Form) umfasst, wobei der IgH-Locus eine humanisierte variable Region zum Exprimieren einer Vielzahl unterschiedlicher menschlicher VH-Domänen umfasst;
3. Einfügen einer Nukleotid-Sequenz, die die Leichtkette des Antikörpers oder das VL davon codiert, in das Genom der ES-Zelle, wobei die Einfügung zwischen dem EiK und dem CK eines Kappa-Locus-Allels des ES-Zellen-Genoms erfolgt, um eine Zelle nach einem der Ansprüche 1-23 zu erzeugen;
4. optional Modifizieren des anderen Kappa-Locus-Allels, sodass es nicht in der Lage ist, Kappa-Leichtketten zu exprimieren, oder Modifizieren des Allels, wodurch eine Nukleotid-Sequenz, die die Leichtkette des ersten oder eines zweiten Antikörpers oder das VL davon codiert, zwischen das EiK und das CK des anderen Kappa-Locus-Allels eingefügt wird;
5. Entwickeln der Es-Zelle in eine Maus, wobei die Maus oder das/die so entwickelte(n) Jungtier(e) mindestens einen Kappa-Locus umfasst/umfassen, der in der Lage ist, die Leichtkette oder das VL zu exprimieren, wobei sich die Leichtkette oder das VL mit Schwerketten oder VH, die/das durch die Maus exprimiert wird/werden, koppelt, um VH/VL-Antigen-Bindungsstellen zu bilden;
6. optional Erlangen von Maus-Nachkommen, wobei die Nachkommen jeweils mindestens einen Kappa-Locus umfassen, der in der Lage ist, die Leichtkette oder das VL zu exprimieren, wobei sich die Leichtkette oder das VL mit Schwerketten oder VH, die/das durch die Maus-Nachkommen exprimiert wird/werden, koppelt, um VH/VL-Antigen-Bindungsstellen zu bilden;
7. Immunisieren der Maus aus Schritt 5 oder eines Maus-Nachkommen mit einem zweiten Antigen, wobei sich das erste und das zweite Antigen unterscheiden;
8. Erlangen einer Nukleotid-Sequenz von einer immunisierten Maus aus Schritt 7, die ein VH (zweites VH) codiert, dass sich mit dem VL koppelt (oder eines VH einer Schwerkette, die sich mit dem VL der Leichtkette koppelt), wobei das zweite VH und VL eine Antigen-Bindungsstelle bilden, die sich spezifisch an das zweite Antigen bindet;
9. Einfügen einer Nukleotidsequenz, die das erste VH codiert, einer Nukleotid-Sequenz, die das zweite VH codiert, und einer Nukleotid-Sequenz, die das VL codiert, in einen oder mehrere Expressionsvektoren oder in das Genom einer oder mehrerer Wirtszellen, wobei jede V-Nukleotid-Sequenz 5' einer ersten CH-, einer zweiten CH- bzw. CL-Region eines Antikörpers zur Expression von ersten Antikörper-Schwerketten, zweiten Antikörper-Schwerketten bzw. Antikörper-Leichtketten in betriebsfähiger Verbindung steht; wobei die erste und die zweite CH-Region optional erste und zweite CH-Domänen codieren, die mutierte menschliche CH-Domänen sind, welche sich miteinander koppeln, und/oder wobei die ersten und die zweiten Ketten unterschiedliches pI aufweisen;
10. Erlangen von Wirtszellen aus Schritt 9 und Exprimieren der Schwer- und Leichtketten in den Wirtszellen, wodurch bispezifische Antikörper erzeugt werden, die jeweils ein erstes Schwerketten-Leichtketten-Paar, das eine VH/VL-Bindungsstelle umfasst, die in der Lage ist, sich spezifisch an das erste Antigen zu binden, und ein zweites Schwerketten-Leichtketten-Paar, das eine VH/VL-Bindungsstelle umfasst, die in der Lage ist, sich spezifisch an das zweite Antigen zu binden, umfassen; und
11. Isolieren der bispezifischen Antikörper.

42. Verfahren zum Erzeugen einer Zelle nach einem der Ansprüche 1 bis 23 oder eines Vertebraten nach einem der Ansprüche 24 bis 30, wobei das Verfahren Folgendes umfasst:
(a) Erlangen einer Nukleinsäure, die eine umgeordnete variable Antikörper-Region umfasst, welche eine umgeordnete Antikörper-V-Domäne codiert; und
(b) Einfügen der variablen Region oder einer Kopie davon in das Genom einer Zelle, wodurch die variable Region in einem Antikörper-Locus in dem Genom enthalten ist,
wobei der Locus (in 5'-3'-Richtung) (c) einen Antikörper-Leichtketten-EiK-Intron-Enhancer;
(d) die umgeordnete variable Antikörper-Region; und
(e) eine konstante Antikörper-Region, die eine Antikörper-Leichtketten-C-Domäne codiert, umfasst;
wobei der Locus dazu betriebsfähig ist, eine Antikörper-Kette zu exprimieren, die (in N-C-Terminus-Richtung) die umgeordnete V-Domäne und die C-Domäne umfasst; und
(f) wobei die Zelle optional eine nichtmenschliche Vertebraten-ES-Zelle oder eine iPS-Zelle ist, wobei das Verfahren ferner Generieren des nichtmenschlichen Vertebraten nach einem der Ansprüche 24 bis 30 aus der Zelle umfasst.

43. Verfahren nach Anspruch 42, wobei die variable Domäne ein VL ist und die C-Domäne CL ist.

44. Verfahren nach Anspruch 42 oder 43, wobei das Einfügen unter Verwendung von homologer Rekombination oder stellenspezifischer Rekombination mit chromosomaler DANN der Zelle ausgeführt wird.

45. Verfahren nach einem der Ansprüche 42 bis 44, umfassend
Erlangen eines Transgens, das (in 5'-3'-Richtung) Folgendes umfasst:
(a) den EiK-Intron-Enhancer;
(b) die umgeordnete variable Antikörper-Region; und
(c) die konstante Antikörper-Leichtketten-Region; und
Einfügen des Transgens in das Genom der Zelle, wodurch der Antikörper-Locus in der Zelle enthalten ist.

46. Verfahren nach einem der Ansprüche 42 bis 44, umfassend Einfügen der variablen Region zwischen
(a) dem Intron-Enhancer und dem CL eines Leichtketten-Locus des Zellgenoms; oder
(b) dem Intron-Enhancer (Eµ) und dem Cu eines Schwerketten-Locus des Zellgenoms.

47. Verfahren nach einem der Ansprüche 42 bis 46, wobei die variable Region eine V-Domäne eines Antikörpers codiert, der sich spezifisch an ein vorbestimmtes Antigen bindet.

48. Verfahren nach Anspruch 47, wobei der Schritt des Erlangens der Nukleinsäure, die die umgeordnete variable Antikörper-Region, wie in Anspruch 42 aufgeführt, umfasst, Folgendes umfasst:
(a) Erlangen einer weiteren Zelle, die den in Anspruch 47 aufgeführten Antikörper exprimiert;
(b) Erlangen oder Kopieren
i. einer DNA-Sequenz der weiteren Zelle, die eine Sequenz der umgeordneten variablen Region umfasst, welche die V-Domäne codiert; oder
ii. einer RNA-Sequenz der weiteren Zelle, die eine Sequenz der umgeordneten variablen Region umfasst, welche die V-Domäne codiert, und Herstellen einer DANN-Kopie der RNA-Sequenz;
wodurch die Nukleinsäure erlangt wird.

49. Verfahren nach einem der Ansprüche 42 bis 48, umfassend Kultivieren der resultierenden Zelle, um eine Vielzahl von Zellen zu erzeugen, wobei jede Zelle eine beliebige nach einem de Ansprüche 1 bis 23 ist.

50. Nichtmenschliche Vertebraten-Blastozyste oder nichtmenschlicher Prä-Morula-Embryo, der in eine ES- oder iPS-Zelle nach einem der Ansprüche 1 bis 23 implantiert oder gemäß dem Verfahren nach einem der Ansprüche 42 bis 49 erlangt wird.

51. Nukleinsäure, umfassend ein Transgen zur Verwendung in dem Verfahren nach Anspruch 45, wobei das Transgen (in 5'-3'-Richtung) Folgendes umfasst:
(a) den EiK-Intron-Enhancer;
(b) die umgeordnete variable Antikörper-Region; und
(c) die konstante Antikörper-Leichtketten-Region.

## Revendications

1. Cellule comprenant un locus de chaîne légère kappa d'anticorps (premier locus), dans laquelle le locus comprend (dans le sens 5' à 3')
(a) un amplificateur intronique EiK de chaîne légère ;
(b) une région variable d'anticorps réarrangée codant pour un domaine V d'anticorps réarrangé ; et
(c) une région constante de chaîne légère d'anticorps codant pour un domaine C de chaîne légère ;
dans laquelle le locus peut être utiliser pour exprimer une chaîne d'anticorps comprenant (dans le sens N- vers C-terminal) ledit domaine V réarrangé et ledit domaine C,
dans laquelle le génome cellulaire comprend un deuxième locus d'anticorps, dans laquelle le deuxième locus est un locus de chaîne lourde d'anticorps non réarrangé comprenant (dans le sens 5' vers 3')
(a) des segments du gène VH ;
(b) un ou plusieurs segments du gène DH ;
(c) un ou plusieurs segments du gène JH ; et
(d) une région constante de chaîne lourde codant pour un ou plusieurs domaines CH ;
dans laquelle le locus de chaîne lourde peut être utilisé pour exprimer une pluralité de chaînes lourdes ; et
dans laquelle ledit locus de chaîne lourde comprend
(i) tous les segments du gène VH humain du groupe constitué de IGHV1-3, IGHV1-8, IGHV1-18, IGHV1-46, IGHV3-7, IGHV3-9, IGHV3-11, IGHV3-15, IGHV3-20, IGHV3-21, IGHV3-23, IGHV3-30, IGHV3-33, IGHV3-48, IGHV3-53, IGHV4-4, IGHV4-31, IGHV4-34, IGHV4-39, IGHV4-59, IGHV4-61, IGHV5-51, IGHV6-1 et IGHV7-4-1 ; ou
(ii) au moins 5 segments du gène VH humain du groupe constitué de IGHV1-3*01, IGHV1-8*01, IGHV1-18*01, IGHV1-46*03, IGHV3-7*01, IGHV3-9*01, IGHV3-11*01, IGHV3-15*01, IGHV3-20*d01, IGHV3-21*03, IGHV3-23*04, IGHV3-30*18, IGHV3-33*01, IGHV3-48*02, IGHV3-53*01, IGHV4-4*02, IGHV4-31*03, IGHV4-34*01, IGHV4-39*01, IGHV4-59*01, IGHV4-61*01, IGHV5-51*01, IGHV6-1*01 et IGHV7-4-1*01 ;

2. Cellule selon une quelconque revendication précédente, dans laquelle
(a) le domaine V réarrangé est un VK et le domaine C est un CK ;
(b) le domaine V réarrangé est un VK et le domaine C est un Cλ ;
(c) le domaine V réarrangé est un vλ et le domaine C est un Cλ ; ou
(d) le domaine V réarrangé est un vλ et le domaine C est un CK.

3. Cellule selon une quelconque revendication précédente, dans laquelle le llocus comprend (dans le sens 5' vers 3')
(a) un promoteur capable de favoriser la transcription de la région variable ;
(b) une séquence nucléotidique codant pour un peptide signal à domaine variable ;
(c) ledit amplificateur intronique ;
(d) ladite région variable d'anticorps réarrangée ; et
(e) ladite région constante de chaîne légère d'anticorps,
dans lequel le locus peut être utilisé pour exprimer des transcrits d'ARN codant (dans le sens N- vers C-terminal) ladite séquence peptidique signal fusionnée à la séquence d'acides aminés dudit domaine variable.

4. Cellule selon une quelconque revendication précédente, dans laquelle la cellule est une cellule ES, iPS, hybridome ou B.

5. Cellule selon une quelconque revendication précédente, dans laquelle le domaine V réarrangé est un domaine V kappa ou lambda.

6. Cellule selon une quelconque revendication précédente, dans laquelle la région variable code pour un domaine V qui a une spécificité de liaison pour un premier antigène prédéterminé ou un premier épitope, dans laquelle le locus peut être utilisé pour exprimer une chaîne d'anticorps qui comprend un domaine V qui conserve ladite spécificité.

7. Cellule selon une quelconque revendication précédente, dans laquelle la cellule comprend un deuxième locus d'anticorps qui peut être utiliser pour exprimer une deuxième chaîne d'anticorps, dans laquelle la deuxième chaîne comprend un deuxième domaine V réarrangé qui forme un site de liaison avec le premier domaine V réarrangé, dans laquelle le site de liaison est capable de se lier spécifiquement à un antigène ou un épitope prédéterminé.

8. Cellule selon la revendication 7 lorsqu'elle dépend de la revendication 6, dans laquelle les antigènes prédéterminés sont différents et lesdits autres et second domaines V sont différents ; ou dans laquelle les épitopes sont différents et lesdits autres et seconds domaines V sont différents.

9. Cellule selon la revendication 8, dans laquelle
(a) le premier domaine est un domaine VL et le second ainsi que lesdits autres domaines V sont des domaines VH ; ou
(b) dans laquelle le premier domaine est un domaine VH et le second ainsi que lesdits autres domaines V sont des domaines VL.

10. Cellule selon une quelconque revendication précédente, dans laquelle la région variable se trouve à moins de 0,5 kb en 3' de l'amplificateur.

11. Cellule selon une quelconque revendication précédente, dans laquelle le locus comprend un deuxième amplificateur qui se trouve en 3' de la région constante.

12. Cellule selon une quelconque revendication précédente, dans laquelle la cellule est une cellule de mammifère non humain, de souris, de rat ou de rongeur.

13. Cellule selon une quelconque revendication précédente, dans laquelle ladite région constante se trouve au niveau d'un locus d'anticorps endogène de la cellule.

14. Cellule selon la revendication 13 lorsqu'elle dépend de la revendication 1, dans laquelle ledit locus endogène est un locus de chaîne kappa endogène.

15. Cellule selon l'une quelconque des revendications 1 à 12, dans laquelle le locus est constitué par un transgène qui est constitué par le génome de la cellule à une position en dehors d'un locus d'anticorps endogène.

16. Cellule selon une quelconque revendication précédente, dans laquelle la cellule est homozygote pour ledit locus.

17. Cellule selon une quelconque revendication précédente lorsqu'elle dépend de la revendication 1, dans laquelle le locus de la chaîne lourde peut être utiliser pour exprimer une pluralité de chaînes lourdes, comprenant une pluralité de spécificités ou d'affinités antigéniques, chacune desdites chaînes lourdes étant capable de s'apparier avec une chaîne d'anticorps codée par le premier locus pour produire des chaînes appariées comprenant un site de liaison à l'antigène.

18. Cellule selon une quelconque revendication précédente, dans laquelle chaque région variable ou segment de gène est humain.

19. Cellule selon une quelconque revendication précédente, dans laquelle la cellule est une cellule de souris et chaque région constante est une région constante de souris, de rat ou humaine.

20. Cellule selon une quelconque revendication précédente, dans laquelle chacun desdits activateurs est un activateur endogène de la cellule.

21. Cellule selon une quelconque revendication précédente, dans laquelle la cellule est une cellule de souris et chacun desdits amplificateurs est un amplificateur de souris.

22. Cellule selon une quelconque revendication précédente, dans laquelle
(a) la cellule est une cellule de souris, la région variable est une région variable humaine, l'amplificateur intronique est un amplificateur intronique de souris au niveau d'un locus d'anticorps endogène de la cellule et ladite région constante est une région constante de souris, de rat ou humaine ; ou
(b) la cellule est une cellule de rat, la région variable est une région variable humaine, l'amplificateur intronique est un amplificateur intronique de rat au niveau d'un locus d'anticorps endogène de la cellule et ladite région constante est une région constante de souris, de rat ou humaine.

23. Cellule selon une quelconque revendication précédente, dans laquelle la région variable réarrangée est un réarrangement de
(a) IGLV3-21 et IGLJ3 humaines et éventuellement connectés de manière fonctionnelle au promoteur de la lignée germinale humaine IGLV3-21 et/ou à une séquence nucléotidique codant pour le peptide signal ;
(b) IGK1-39 et IGKJ1 humaines ou 5 et éventuellement connectées de manière opérationnelle au promoteur de la lignée germinale humaine IGKV1-39 et/ou à la séquence nucléotidique codant pour le peptide signal ;
(c) IGK3-20 et IGKJ1 humaines ou 5 et éventuellement connectées de manière opérationnelle au promoteur de la lignée germinale humaine IGKV3-20 et/ou à la séquence nucléotidique codant pour le peptide signal ;
(d) une VpreB et une J\5 humaines et éventuellement connectés de manière fonctionnelle au promoteur VpreB de la lignée germinale humaine et/ou à une séquence nucléotidique codant pour le peptide signal.

24. Vertébré non humain transgénique comprenant une pluralité de cellules selon une quelconque revendication précédente.

25. Vertébré selon la revendication 24, dans lequel la lignée germinale dudit vertébré comprend un premier locus (et éventuellement le second locus) tel que défini dans l'une quelconque des revendications 1 à 23.

26. Vertébré selon la revendication 24, dans lequel le vertébré est une chimère desdites cellules et d'une pluralité d'autres cellules qui ne comprennent pas le premier locus, dans lequel la lignée germinale du vertébré ne comprend pas ledit premier locus.

27. Vertébré selon l'une quelconque des revendications 24 à 26, comprenant une première pluralité de cellules et une deuxième pluralité de cellules, dans laquelle les cellules de la première pluralité comprennent le même premier locus d'anticorps identique, et les cellules de la deuxième pluralité comprennent le même locus d'anticorps identique, dans lequel chacun desdits locus d'anticorps est conforme au premier locus de l'une quelconque des revendications 1 à 27,
dans lequel le vertébré est capable d'exprimer une première pluralité de chaînes d'anticorps provenant dudit premier locus identique et une seconde pluralité de chaînes d'anticorps provenant dudit locus supplémentaire, et dans lequel le locus anticorps des premières cellules est différent du locus d'anticorps des secondes cellules.

28. Vertébré selon la revendication 27, dans lequel le vertébré comprend une troisième pluralité de cellules, dans laquelle les cellules de la troisième pluralité comprennent le même autre locus d'anticorps, dans lequel l'autre locus est conforme à l'une quelconque des revendications 1 à 23 et est différent desdits loci d'anticorps des première et deuxième cellules, dans laquelle le vertébré est capable d'exprimer une troisième pluralité de chaînes d'anticorps à partir de l'autre locus.

29. Vertébré selon la revendication 27 ou 28, dans lequel le vertébré est une chimère desdites cellules et d'une pluralité d'autres cellules qui ne comprennent pas un premier locus selon l'une quelconque des revendications 1 à 24 ou lesdits locus supplémentaires ou autres.

30. Vertébré selon l'une quelconque des revendications 24 à 29, dans lequel le répertoire de chaînes légères d'anticorps est pur à au moins 99 % pour une seule espèce de domaine VL réarrangé.

31. Population de cellules de rate, de moelle osseuse, de cellules B, d'hybridomes, de cellules CHO, de cellules HEK, de cellules MEF, de cellules COS, de cellules HeLa ou de cellules sanguines, dans laquelle chaque cellule est conforme à l'une quelconque des revendications 1 à 23 ; et facultativement, dans laquelle les cellules expriment au moins 10 espèces d'anticorps différentes, dans laquelle
(a) au moins 95 % des anticorps partagent le même domaine VL de chaîne légère et la population comprend au moins 10 espèces de domaine VH différentes ; ou
(b) les anticorps comprennent des domaines VL dérivés de la recombinaison d'un premier segment de gène VL et d'un premier segment de gène JL, dans laquelle au moins 95 % de tous lesdits domaines VL dérivés de ladite recombinaison comprennent la même séquence d'acides aminés VL et la population comprend au moins 10 espèces de domaine VH différentes.

32. Procédé d'identification ou d'obtention d'un anticorps, d'un domaine variable d'anticorps, d'une séquence nucléotidique codant pour un anticorps ou d'un domaine variable d'anticorps, ou d'un vecteur d'expression ou d'une cellule hôte qui est capable d'exprimer l'anticorps ou le domaine, dans lequel l'anticorps ou le domaine est capable de se lier spécifiquement à un antigène cible, le procédé comprenant
(a) la mise en contact de la population de cellules selon la revendication 31 avec ledit antigène ;
(b) la liaison des anticorps exprimés ou compris par ladite population audit antigène ; et
(c) l'isolation ou l'identification d'un ou de plusieurs anticorps qui se lient à l'antigène, ou isolation ou identification d'un domaine VH et/ou VL dudit ou desdits anticorps ; ou l'identification d'une séquence nucléotidique codant pour ledit VH ou VL ; et
(d) éventuellement
(i) la corrélation dudit anticorps ou domaine identifié avec une séquence nucléotidique codant pour celui-ci, identifiant ainsi ladite séquence ;
(ii) l'amplification de ladite séquence et l'insertion de la séquence dans un vecteur d'expression ou un génome de cellule hôte pour l'expression de l'anticorps ou du domaine codé ; et
(iii) éventuellement l'expression et l'isolation dudit anticorps ou domaine,
dans lequel les étapes (i) et (ii) peuvent être réalisées dans n'importe quel ordre.

33. Procédé selon la revendication 32, comprenant l'utilisation du procédé pour amplifier les séquences nucléotidiques VH et VL codant pour les domaines VH et VL de l'anticorps identifié et comprenant en outre l'insertion des séquences nucléotidiques VH et VL dans le vecteur ou la cellule pour la co-expression du VH et du VL pour produire des sites de liaison VH/VL appariés qui sont capables de se lier à l'antigène et d'exprimer (et éventuellement d'isoler) les sites de liaison.

34. Procédé selon la revendication 33, dans lequel le procédé comprend l'expression du VH et du VL en présence d'une autre espèce de domaine VH, dans lequel le VL et l'autre VH forment des sites VH/VL supplémentaires appariés qui sont capables de se lier à un autre antigène, dans lequel l'autre antigène et l'antigène mentionné dans la revendication 33 sont différents, le procédé comprenant la co-expression (et éventuellement l'isolement) des sites de liaison, dans lequel les sites de liaison sont constitués d'anticorps bispécifiques codés par le vecteur ou la cellule, dans lequel les anticorps bispécifiques comprennent un site de liaison respectif pour chacun desdits antigènes.

35. Procédé selon la revendication 34, dans lequel les anticorps bispécifiques sont des anticorps à 4 chaînes comprenant une première paire chaîne lourde-chaîne légère et une seconde paire chaîne lourde-chaîne légère, dans lequel
(a) la chaîne légère dans les première et seconde paires comprend ledit domaine VL,
(b) la chaîne lourde de la première paire comprend le domaine VH selon la revendication 34, dans lequel le domaine VH forme un site de liaison VH/VL avec ledit domaine VL et dans lequel le site de liaison est capable de se lier spécifiquement audit antigène selon la revendication 33 ;
(c) la chaîne lourde de la seconde paire comprend ledit autre domaine VH qui forme un site de liaison VH/VL avec ledit domaine VL et dans lequel le site de liaison est capable de se lier spécifiquement audit autre antigène selon la revendication 34.

36. Procédé selon la revendication 35, dans lequel chaque anticorps à 4 chaînes comprend des régions constantes de chaîne lourde mutées pour favoriser l'appariement des chaînes lourdes des première et seconde paires lourdes-légères ; éventuellement dans lequel les mutations sont des mutations knob-in-hole ou des mutations de paires de charges.

37. Procédé selon l'une quelconque des revendications 32 à 36, dans lequel le procédé comprend l'obtention de séquences nucléotidiques codant pour VL et/ou VH à partir des cellules de la revendication 31 ou 35, et à l'étape (d) (i) l'utilisation des séquences nucléotidiques pour corréler une ou plusieurs d'entre elles avec un ou plusieurs domaines VH et/ou VL desdits anticorps qui se lient à l'antigène.

38. Procédé d'obtention d'une séquence nucléotidique codant pour un anticorps ou un domaine variable d'anticorps, dans lequel l'anticorps ou le domaine est capable de se lier spécifiquement à un antigène cible, le procédé comprenant
(a) l'obtention des séquences nucléotidiques codant pour VL et/ou VH à partir des cellules selon la revendication 31 ou 35 ;
(b) l'amplification de ladite séquence ; et
(c) éventuellement l'insertion de la séquence dans un vecteur d'expression ou un génome de cellule hôte pour l'expression de l'anticorps ou du domaine codé.

39. Procédé d'obtention d'une lignée cellulaire productrice d'anticorps pour la production d'anticorps qui se lient spécifiquement à un antigène, le procédé comprenant l'obtention de séquences nucléotidiques codant pour VL et/ou VH à partir des cellules selon la revendication 31 ou 35 ; et l'insertion des séquences dans le génome d'une cellule hôte, dans lequel
(a) chaque séquence VH est insérée de manière opérationnelle en 5' d'une région constante de chaîne lourde d'anticorps pour l'expression de chaînes lourdes par la cellule comprenant les domaines VH et C ;
(b) chaque séquence VL est insérée de manière opérationnelle en 5' d'une région constante de chaîne légère d'anticorps pour l'expression de chaînes légères par la cellule comprenant les domaines VL et C ;
(c) dans lequel les chaînes lourdes exprimées sont capables de s'apparier aux chaînes légères pour produire des paires de chaînes lourdes-légères, chaque paire comprenant un site de liaison VH/VL qui est capable de se lier à un antigène ; et
(d) dans lequel les séquences nucléotidiques codant pour VH et VL sont les séquences de séquences nucléotidiques codant pour VH et VL d'une ou plusieurs cellules selon la revendication 31.

40. Procédé selon la revendication 39, comprenant l'insertion d'une première et d'une seconde séquences nucléotidiques codant pour VH dans le génome cellulaire, dans lequel
(a) le premier VH (lorsqu'il est exprimé comme faisant partie des premières chaînes lourdes) est capable de s'apparier au VL (lorsqu'il est exprimé comme faisant partie de chaînes légères) pour former un premier site de liaison VH/VL capable de se lier spécifiquement à un premier antigène ;
(b) le second VH (lorsqu'il est exprimé comme partie de deuxièmes chaînes lourdes) est capable de s'apparier au VL (lorsqu'il est exprimé comme partie de chaînes légères) pour former un second site de liaison VH/VL qui est capable de se lier spécifiquement à un second antigène ; et
(c) les premier et second antigènes sont différents ;
dans lequel les première et seconde chaînes lourdes et les chaînes légères sont capables de s'apparier pour produire des anticorps bispécifiques à 4 chaînes comprenant une première paire chaîne lourde-chaîne légère et une seconde paire chaîne lourde-chaîne légère, dans lequel
(d) la chaîne lourde de la première paire chaîne lourde-chaîne légère comprend le premier VH ; et
(e) la chaîne lourde de la seconde paire chaîne lourde-chaîne légère comprend le second domaine VH.

41. Procédé d'obtention d'un anticorps bispécifique, le procédé comprenant :
1. l'obtention d'un premier anticorps comprenant des premiers domaines VH et VL humains, dans lequel l'anticorps se lie spécifiquement à un premier antigène prédéterminé ; ou l'obtention des séquences nucléotidiques codant pour ledit VL ou la chaîne légère de l'anticorps ;
2. l'obtention d'une cellule ES de souris transgénique comprenant un locus IgH (sous forme hétérozygote ou homozygote), dans lequel le locus IgH comprend une région variable humanisée pour exprimer une pluralité de domaines VH humains différents ;
3. l'insertion d'une séquence nucléotidique codant pour la chaîne légère de l'anticorps ou la VL de celui-ci dans le génome de la cellule ES, dans lequel l'insertion se fait entre EiK et CK d'un allèle du locus kappa du génome de la cellule ES pour produire une cellule selon l'une quelconque des revendications 1 à 23 ;
4. la modification éventuelle de l'autre allèle du locus kappa de sorte qu'il ne soit pas capable d'exprimer des chaînes légères kappa, ou la modification de l'allèle moyennant en quoi une séquence nucléotidique codant pour la chaîne légère du premier ou d'un second anticorps ou le VL de celui-ci est insérée entre l'EiK et la CK dudit autre allèle du locus kappa ;
5. le développement de la cellule ES chez une souris, dans lequel la souris ou le ou les chiots ainsi développés comprennent au moins un locus kappa qui est capable d'exprimer la chaîne légère ou VL, dans lequel la chaîne légère ou VL s'apparie à des chaînes lourdes ou VH exprimées par la souris pour former des sites de liaison à l'antigène VH/VL ;
6. l'obtention éventuelle d'une descendance de souris, dans lequel la descendance comprend chacune au moins un locus kappa qui est capable d'exprimer la chaîne légère ou VL, dans lequel la chaîne légère ou VL s'apparie à des chaînes lourdes ou VH exprimées par la descendance de souris pour former des sites de liaison à l'antigène VH/VL ;
7. l'immunisation de la souris de l'étape 5 ou ladite descendance de souris avec un second antigène, dans lequel le premier et le second antigène sont différents ;
8. l'obtention à partir d'une souris immunisée de l'étape 7 d'une séquence nucléotidique codant pour un VH (second VH) qui s'apparie avec le VL (ou un VH d'une chaîne lourde qui s'apparie avec le VL de la chaîne légère), dans lequel le second VH et le VL forment un site de liaison à l'antigène qui se lie spécifiquement au second antigène ;
9. l'insertion d'une séquence nucléotidique codant pour le premier VH, d'une séquence nucléotidique codant pour le second VH et d'une séquence nucléotidique codant pour le VL dans un ou plusieurs vecteurs d'expression ou dans le génome d'une ou de plusieurs cellules hôtes, chaque séquence nucléotidique V étant en connexion fonctionnelle en 5' d'une première région d'anticorps CH, une seconde région CH ou CL d'anticorps respectivement pour l'expression de premières chaînes lourdes d'anticorps, de secondes chaînes lourdes d'anticorps ou de chaînes légères d'anticorps respectivement ; éventuellement, dans lequel les première et seconde régions CH codent pour les premier et second domaines CH qui sont des domaines CH humains mutés qui s'apparient ensemble et/ou dans lequel les première et seconde chaînes ont des pl différents ;
10. l'obtention de cellules hôtes de l'étape 9 et l'expression des chaînes lourdes et légères dans les cellules hôtes, moyennant quoi des anticorps bispécifiques sont produits, chacun comprenant une première paire chaîne lourde/chaîne légère comprenant un site de liaison VH/VL qui est capable de se lier spécifiquement au premier antigène, et une seconde paire chaîne lourde/chaîne légère comprenant un site de liaison VH/VL qui est capable de se lier spécifiquement au second antigène ; et
11. l'isolation desdits anticorps bispécifiques.

42. Procédé de production d'une cellule selon l'une quelconque des revendications 1 à 23 ou vertébré selon l'une quelconque des revendications 24 à 30, le procédé comprenant
(a) l'obtention d'un acide nucléique comprenant une région variable d'anticorps réarrangée codant pour un domaine V d'anticorps réarrangé ; et
(b) l'insertion de la région variable ou d'une copie de celle-ci dans le génome d'une cellule, moyennant quoi la région variable est constituée par un locus d'anticorps dans ledit génome,
dans lequel le locus comprend (dans le sens 5' vers 3') (c)un amplificateur intronique EiK de chaîne légère d'anticorps ;
(d) ladite région variable d'anticorps réarrangée ; et
(e) une région constante d'anticorps codant pour un domaine C de chaîne légère d'anticorps ;
dans lequel le locus peut être utiliser pour exprimer une chaîne d'anticorps comprenant (dans le sens N- vers C-terminal) ledit domaine V réarrangé et ledit domaine C ; et
(f) éventuellement, dans lequel la cellule est une cellule ES de vertébré non humain ou une cellule iPS, le procédé comprenant en outre la génération du vertébré non humain selon l'une quelconque des revendications 24 à 30 à partir de la cellule.

43. Procédé selon la revendication 42, dans lequel le domaine variable est un VL et le domaine C est un CL.

44. Procédé selon la revendication 42 ou 43, dans lequel ladite insertion est réalisée en utilisant une recombinaison homologue ou une recombinaison spécifique à un site avec l'ADN chromosomique de la cellule.

45. Procédé selon l'une quelconque des revendications 42 à 44, comprenant
l'obtention d'un transgène comprenant (dans le sens 5' vers 3')
(a) ledit amplificateur intronique EiK ;
(b) ladite région variable d'anticorps réarrangée ; et
(c) ladite région constante de chaîne légère d'anticorps ; et
l'insertion dudit transgène dans le génome de la cellule, moyennant quoi ledit locus d'anticorps est compris par la cellule.

46. Procédé selon l'une quelconque des revendications 42 à 44, comprenant l'insertion de ladite région variable entre
(a) l'amplificateur intronique et le CL d'un locus de chaîne légère du génome cellulaire ; ou
(b) l'amplificateur intronique (Eµ) et le Cµ d'un locus de chaîne lourde du génome cellulaire.

47. Procédé selon l'une quelconque des revendications 42 à 46, dans lequel la région variable code pour un domaine V d'un anticorps qui se lie spécifiquement à un antigène prédéterminé.

48. Procédé selon la revendication 47, dans lequel l'étape d'obtention de l'acide nucléique comprend la région variable d'anticorps réarrangée selon la revendication 42 comprend
(a) l'obtention d'une autre cellule qui exprime ledit anticorps selon la revendication 47 ;
(b) l'obtention ou la copie
i. d'une séquence d'ADN de l'autre cellule qui comprend une séquence de région variable réarrangée codant pour le domaine V ; ou
ii. d'une séquence NA de l'autre cellule qui comprend une séquence de région variable réarrangée codant pour le domaine V, et créant une copie d'ADN de ladite séquence d'ARN ;
obtenant ainsi ledit acide nucléique.

49. Procédé selon l'une quelconque des revendications 42 à 48, comprenant la culture de la cellule résultante pour produire une pluralité de cellules, dans lequel chaque cellule est selon l'une quelconque des revendications 1 à 23.

50. Blastocyste ou embryon pré-morula de vertébré non humain implanté avec une cellule ES ou iPS selon l'une quelconque des revendications 1 à 23 ou obtenu par le procédé selon l'une quelconque des revendications 42 à 49.

51. Acide nucléique comprenant un transgène destiné à être utilisé dans le procédé selon la revendication 45, le transgène comprenant (dans le sens 5' vers 3')
(a) ledit amplificateur intronique EiK ;
(b) ladite région variable d'anticorps réarrangée ; et
(c) ladite région constante de chaîne légère d'anticorps.
